# EUROPEAN PATENT SPECIFICATION

(11) **EP 3 359 012 B1**
(45) Date of publication and mention of the grant of the patent: **24.08.2022**
(21) Application number: 16853133.3
(22) Date of filing: 07.10.2016
(51) Int. Cl.: A61B 1/313, A61B 1/04, A61B 1/06, A61B 17/00

(54) **A LAPAROSCOPIC TOOL SYSTEM FOR MINIMALLY INVASIVE SURGERY**
LAPAROSKOPISCHES INSTRUMENTENSYSTEM FÜR MINIMAL-INVASIVE CHIRURGIE
SYSTÈME D'OUTILS LAPAROSCOPIQUE POUR CHIRURGIE À EFFRACTION MINIMALE

(30) Priority: 09.10.2015 DK 201570642; 14.06.2016 DK 201670428
(43) Date of publication of application: 15.08.2018
(73) Proprietor: 3DIntegrated ApS, 2200 Copenhagen N (DK)
(72) Inventor: HANSEN, André, 2200 Copenhagen N (DK); KIRKEGAARD, Henriette Schultz, 1757 Copenhagen V (DK); HANSEN, Steen Møller, 8541 Skødstrup (DK)
(74) Representative: Plougmann Vingtoft a/s
(86) International application number: PCT/DK2016/050327
(87) International publication number: WO 2017/059870

(56) References cited:
- EP-A1- 2 412 290
- EP-A1- 2 551 698
- US-A- 5 769 791
- US-A1- 2009 054 767
- US-A1- 2013 162 775
- US-A1- 2013 281 845
- US-A1- 2015 119 637

## Description

### TECHNICAL FIELD

The invention relates to a laparoscopic tool system for minimally invasive surgery, the laparoscopic tool system comprises a laparoscopic tool adapted for use during a minimally invasive surgery procedure and/or a minimally invasive examination procedure.

### BACKGROUND ART

Minimally invasive surgery (MIS) and in particular laparoscopy has been used increasingly in recent years due to the benefits compared to conventional open surgery as it reduces the trauma to the patient tissue, leaves smaller scars, minimizes post-surgical pain and enables faster recovery of the patient.

There are different kinds of MIS such as laparoscopy, endoscopy, arthroscopy and thoracoscopy. Whereas many of the MIS procedures are mainly for examination within natural openings of mammals, laparoscopy has in recent years developed to be a preferred method of performing both examination and surgical procedures.

In laparoscopic surgery the surgeon accesses a body cavity, such as the abdominal or pelvic cavity, through a series of small incisions. A laparoscope is inserted through an incision, and conventionally connected to a monitor, thereby enabling the surgeon to see the inside of the abdominal or pelvic cavity. In order to perform the surgical procedure, surgical instruments are inserted through other incisions. In addition, the body cavity (surgery cavity) around the surgical site is inflated with a fluid, preferably gas e.g. carbon dioxide in order to create an 'air' space within the cavity to make space for the surgeon to view the surgical site and move the laparoscopic instruments.

Minimally invasive surgery is generally performed through openings in a patient's skin - often relatively small openings - and the surgical site is visualized for the surgeon by inserting a laparoscope which comprises illumination means and a camera into the body cavity and displaying the images on a screen.

In order to improve the vision for the surgeon, in particular to make it easier for the surgeon to determine the sizes of various organs, tissues, and other structures in a surgical site, several in-situ surgical metrology methods have been provided in the prior art. Different types of optical systems have been applied to provide an improved vision of the surgical site. Some prior art systems are focused on providing 3D images of the MIS cavity and other prior art systems are focused on monitoring the position of a laparoscopic tool.

US 2013/0296712 describes an apparatus for determining endoscopic dimensional measurements, including a light source for projecting light patterns on a surgical sight including shapes with actual dimensional measurements and fiducials, and means for analyzing the projecting light patterns on the surgical site by comparing the actual dimensional measurements of the projected light patterns to the surgical site.

WO 2013/163391 describes at system for generating an image, which the surgeon may use for measuring the size of or distance between structures in the surgical field by using an invisible light for marking a pattern to the surgical field. The system comprises a first camera; a second camera; a light source producing light at a frequency invisible to the human eye; a dispersion unit projecting a predetermined pattern of light from the invisible light source; an instrument projecting the predetermined pattern of invisible light onto a target area; a band pass filter directing visible light to the first camera and the predetermined pattern of invisible light to the second camera; wherein the second camera images the target area and the predetermined pattern of invisible light, and computes a three-dimensional image.

US2008071140 discloses an endoscopic surgical navigation system which comprises a tracking subsystem to capture data representing positions and orientations of a flexible endoscope during an endoscopic procedure, to allow co-registration of live endoscopic video with intra-operative and/or preoperative scan images. Positions and orientations of the endoscope are detected using one or more sensors and/or other signal-producing elements disposed on the endoscope.

US2010268067 discloses methods, systems, devices, and computer-readable media for image guided surgery for allowing a physician to use multiple instruments for a surgery and simultaneously provide image-guidance data for those instruments.

US2011069159 discloses a system for orientation assistance and display of an instrument that is inserted or present in the natural or artificially produced hollow cavity (human, animal, object), and that is equipped with one or more sensor units. Multiple measurements of the 3D position of the instrument equipped with one or more sensor units are performed by positioning a measuring system, so that a precise orientation and positioning of the instrument in the body may be computed. The 3D position data are used to compute a virtual image of the instrument synchronously. The virtual images are then either projected directly in exact position onto the body surface of a person or combined in a body surface image (real video camera image of the patient) onto a monitor or superimposed (virtual or augmented reality).

It has also been suggested to generate augmented reality vision of surgery cavities for providing an improved view of internal structures of the organs of a patient to determine the minimal distance to a cavity surface or organ of a patient. Such systems are described in the articles "Augmented reality in laparoscopic surgical oncology" by Stéphane Nicolau et al. Surgical Oncology 20 (2011) 189-201 and "An effective visualization technique for depth perception in augmented reality-based surgical navigation" by Choi Hyunseok et al. The international journal of medical robotics and computer assisted surgery, 2015 May 5. doi: 10.1002/rcs.1657.

US2013162775 , which is the document disclosing the features of the preample of claim 1, discloses an apparatus for endoscopic 3D data collection, comprising a light generator to generate at least a modulated measuring radiation, a light transmitter to transmit the measuring radiation to at least one partial area of a surface of an internal bodily cavity, which is at least partly situated in an endoscopically insertable elongated shaft, an observation lens situated in a distal end portion of the shaft to receive a signal radiation from at least the partial area of the surface of the cavity, an image transmitter situated at least partly inside the shaft to transmit the signal radiation from the distal to a proximal end portion of the shaft for reception by a time-of-flight image sensor and a controller to control the light generator, to control the time-of-flight image sensor, and to evaluate data supplied by the time-of-flight image sensor to generate 3D data, wherein the apparatus includes a position sensor to capture a position and an orientation of the shaft.

EP2551698 discloses an instrument with acceleration sensors for detecting a position change of a field of view of an optical sensor. An image processing unit is designed such that output signals of the optical sensor and output signals of the acceleration sensors are processed together, so that image information of the optical sensor in different positions of the field of view are combined to form a three-dimensional overall image of an object based on the position of the field of view. A radiation source emits a modulated radiation towards the field of view. The optical sensor is designed as a video sensor.

US2009054767 discloses a device comprising: a first light source configured to emit light onto a region of interest; a light detector; and a visible light source configured to emit visible light onto the region of interest. The visible light has a color that is selected based on the amount of light detected by the light detector.

US5769791 discloses an apparatus for non-destructive interrogation of tissue comprising: a minimally invasive surgical tool end effector; a first element positioned at the end effector to launch one or more discrete wavelengths of radiation into a tissue to be interrogated; a second element positioned at the end effector to couple light launched from the first element that has passed through the tissue to be interrogated; a photodetector operatively coupled to said second element having an output of electrical signals corresponding to at least a portion of the light coupled by the second element; and a processor having an input for receiving said output of electrical signals: The processor is responsive to the output of electrical signals to determine non-destructively at least one characteristic of the tissue to be interrogated.

### DISCLOSURE OF INVENTION

The object of the present invention is to provide an alternative system for providing good real life visibility of at least a part of a body cavity during a laparoscopic minimally invasive surgery to provide accurate real life information to the operator of a laparoscopic tool about spatial shape and/or positions and/or surface area information to thereby make it simpler for the operator to manipulate the laparoscopic tool during a laparoscopic procedure and/or for simpler examination of a surface area within a surgery cavity.. Thereby the laparoscopic procedure may be performed faster and/or with reduced risk of unintended damage to the patient.

This and other objects have been solved by the invention or embodiments thereof as defined in the claims and as described herein below.

It has been found that the invention or embodiments thereof have a number of additional advantages, which will be clear to the skilled person from the following description.

The invention is defined in claim 1.

In an embodiment the laparoscopic tool system for minimally invasive surgery additionally comprises a time-of-flight distance sensor set, comprising a time-of-flight light emitter and a time-of-flight receiver.

The laparoscopic tool system has been found to ensure a very accurate real time distance and/or spatial information to the operator during a minimally invasive surgery procedure. Even where smoke, fog or similar which usually results in blurring up images obtained by an camera, such as a stereo camera is within the minimally invasive surgery cavity, it has been found that the laparoscopic tool system of the invention still may operate with a very high accuracy.

The terms "distal" and "proximal" should be interpreted in relation to the orientation of the laparoscopic tool, such as a laparoscopic instrument i.e. the distal end of the laparoscopic tool is the part of the laparoscopic tool furthest from a handle or collar portion which constitutes the proximal end of the laparoscopic tool.

The phrase "distal to" means "arranged at a position in distal direction to the laparoscopic tool, where the direction is determined as a straight line between a proximal end of the laparoscopic tool and the distal end of the laparoscopic tool. The phrase "distally arranged" means arranged distal to the distal end of the laparoscopic tool.

The phrase "real time" is herein used to mean the time it requires the computer to receive and process constantly changing data optionally in combination with other data, such as predetermined data, reference data, estimated data which may be non-real time data such as constant data or data changing with a frequency of above 1 minute to return the real time information to the operator. "Real time" may include a short delay, such as up to 5 seconds, preferably within 1 second, more preferably within 0.1 second of an occurrence.

The Term "operator" is used to designate a surgeon or a robotic surgeon i.e. a robot programmed to perform a laparoscopic procedure on a patient.

The term "access port" means a port through which a surgical instrument may be inserted. The access port may comprise a seal or an insulation, a lid and/or similar which fully or partly locks or fills out the access port when the laparoscopic instrument is not inserted in the access port. The seal, insulation and/or seal ensure that undesired amounts of gasses do not escape and deflate the body cavity. When a laparoscopic tool is not inserted in the access port, the seal or the insulation advantageously seals against undesired leakage of gas.

The term "rigid connection" means a connection which ensures that the relative position between rigidly connected elements is substantially constant during normal use.

The term "laparoscopic tool " means herein any tool adapted for being inserted into a small surgical incision in the mammal skin e.g. the abdominal wall. The laparoscopic tool includes a laparoscopic instrument, a laparoscope, a penetrator and a cannula.

Laparoscopic tools comprise dynamic laparoscopic tools and static laparoscopic tool. Static laparoscopic tools include laparoscopes (sometimes referred to as endoscopes), penetrators and cannulas. Static laparoscopic tools are generally held in stationary position during the actual minimally invasive surgery procedure or in the case of a laparoscopic tool in the form of a penetrator. A penetrator is usually applied for making the incision to provide an access port for a dynamic laparoscopic tool. A penetrator is often in the form of an obturator or a trocar. A Trocar comprises an obturator and a cannula. Upon incision by the obturator the cannula is positioned to form an access port to the MIS cavity. The obturator may have a channel for feeding gas to form or to expand the minimally invasive surgery cavity, where after the obturator is removed, leaving the cannula as an access port.

Dynamic laparoscopic tools includes laparoscopic instruments, Such laparoscopic instrument are used for dynamic movements within the minimally invasive surgery cavity for performing the actual minimally invasive surgery procedure.

The term "laparoscopic instrument" means herein a laparoscopic tool comprising a surgical tool adapted for performing surgery onto the tissue within the minimally invasive surgery cavity e.g. a grasper, a suture grasper, a cutter, a sealer, a stapler, a clip applier, a dissector, scissors, shears, a suction instrument, a clamp instrument, an electrode, a coagulation device, a curette, ablators, scalpels, a needle holder, a needle driver, a spatula, forceps, a biopsy and retractor instrument or a combination thereof.

As explained above a dynamic laparoscopic tool is a laparoscopic instrument.

A laparoscopic instrument comprising a grasper as its surgical tool for performing surgery is referred to as a grasper instrument and etc. The surgical tool may in an embodiment be detachable from the remaining part of the laparoscopic instrument.

In an embodiment the laparoscopic instrument is selected from graspers, scissors or staplers, preferably the laparoscopic instrument is adapted for performing a surgical procedure on the surface area, e.g. comprising direct interaction with tissue at or in the vicinity of the tissue surface area.

The laparoscopic instrument is not an incision instrument such as a penetrator or an obturator. Advantageously the surgical tool if the laparoscopic instrument does not have any sharp edge for cutting through mammal skin.

Preferably the laparoscopic instrument in adapted for being inserted into the minimally invasive surgery cavity via a cannula access port and manipulated within the surgical field distal from the cannula access port or port of entry with the surgical cavity said surgical field.

The dynamic laparoscopic tool is advantageously configured for being dynamically moved within the minimally invasive surgery cavity at a position distal relative to the access port and/or the cannula.

The term "cannula" means herein a hollow tool adapted for being inserted into an incision to provide an access port as defined above.

The term "laparoscope" means herein a laparoscopic tool, which is not a laparoscopic instrument or a cannula. A laparoscope will usually carry a light receiver, a camera, an emitter, an illuminator or any combinations thereof.

The term "substantially" should herein be taken to mean that ordinary product variances and tolerances are comprised.

The term "about" is generally used to include what is within measurement uncertainties. When used in ranges the term "about" should herein be taken to mean that what is within measurement uncertainties is included in the range.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

The laparoscopic tool system is particularly focused on generating 3D imaging of at least a surface area of the surgery cavity to increase the visual perception of the operator. The laparoscopic tool system of the has been found not only to increase the visual perception but also to provide in real life image scanning with a high flexibility because the system is focused to generate real life 3D imaging on a surface area which is changing as the laparoscopic tool is moved so that a relevant surface area at any time automatically may be subjected to the 3D imaging.

The laparoscopic tool system of the is therefore highly suitable for performing laparoscopic examination procedures

This flexibility of the 3D imaging may result in an even faster laparoscopic procedure and with even higher accuracy.

In an embodiment of the laparoscopic tool system, the time-of-flight image sensor is fixed to the distal portion of the laparoscopic tool, the spatial position tracking means is adapted for tracking the spatial position of the time-of-flight image sensor and generating real time spatial position data of the time-of-flight image sensor.

Advantageously the time-of-flight light emitter is adapted to emitting light and the time-of-flight image sensor is adapted to sensing light from the time-of-flight emitter and generating sensed light data when light is reflected from a surface area within a preselected distance from the time-of-flight image sensor.

The computer system is in data communication with the spatial position tracking means for receiving real time spatial position data of the time-of-flight image sensor. The computer system is also in data communication with the time-of-flight camera set to receiving the sensed light data timely corresponding to the real time spatial position data of the time-of-flight image sensor. The computer system is programmed to calculate the 3D image(s) based on the sensed light data and real time spatial position data of the time-of-flight image sensor.

It is preferred that the computer system is programmed to calculate real life 3D images based on the sensed light data and real time spatial position data of the time-of-flight image sensor.

The laparoscopic tool is a tool which is adapted to being moved during the minimally invasive surgery i.e. at least the distal portion of the laparoscopic tool is adapted to being moved, preferably at least by lateral movements. The laparoscopic tool is preferably selected from a cannula, a laparoscope and a laparoscopic instrument.

In an embodiment, the laparoscopic tool is a cannula as described in DK PA 2015 70483 with the modification that the cannula comprises a time-of-flight image sensor at its distal portion.

The spatial position tracking means may be any means suitable for tracking the position of the distal portion of the time-of-flight image sensor and thereby the laparoscopic tool. The spatial position tracking means is configured for tracking the distal portion of the laparoscopic tool and based on this determine the spatial position of the time-of-flight image sensor.

The spatial position tracking means is adapted for tracking the spatial position of the time-of-flight image sensor relative to a fixed point, a preselected point, and/or relative to an X,Y,Z coordinate matrix.

In an embodiment the spatial position tracking means is advantageously adapted for tracking the spatial position of the time-of-flight image sensor relative to a selected spatial start position. The selected spatial start position may be selected by a user - e.g. via a user interface and/or it may be selected from a database comprising suitable spatial start positions. In an embodiment the operator may be guided to position the laparoscopic tool such that the time-of-flight image sensor is in a selected spatial start position.

The X,Y,Z coordinate matrix may be a spatial reference system, such as a Spatial Reference System Identifier (SRID) system such as those known from spatially enabled databases (such as IBM DB2, IBM Informix, Microsoft SQL Server, MySQL, Oracle, Teradata, PostgreSQL and SQL Anywhere).

In an embodiment the X,Y,Z coordinate matrix may be a local matrix e.g., generated from a number of spatially arranged sensors.

In an embodiment, the spatial position tracking means is adapted to tracking the spatial position of the time-of-flight image sensor relative to the time-of-flight light emitter.

In an embodiment, the spatial position tracking means comprises at least one sensor mounted to the distal portion of the laparoscopic tool.

In an embodiment, the position tracking means is adapted for tracking positions and orientations of the time-of-flight image sensor by a using one or more sensors and/or other signal-producing elements disposed on the laparoscopic tool, preferably immediately adjacent to the time-of-flight image sensor or optionally integrated with the time-of-flight image sensor.

In an embodiment, the spatial position tracking means comprises a motion sensor adapted for determining motions of the laparoscopic tool.

In an embodiment, the position tracking means comprises an inertial navigation system. The inertial navigation system advantageously includes at least a computer (e.g. of the computer system) and a platform or module containing accelerometers, gyroscopes and/or other motion-sensing devices. The inertial navigation system may initially be provided with its position and velocity from another source (such as a human operator, a GPS satellite receiver, etc.), and thereafter computes its own updated position and velocity by integrating information received from the motion sensors. The advantage of an inertial navigation system is that it requires no external references in order to determine its position, orientation, or velocity once it has been initialized.

In an embodiment, the position tracking means comprising a sensor element adapted to be or being physically connected to or integrated with the distal portion of the laparoscopic tool.

In an embodiment, the position tracking means comprises a magnetic motion capture system comprising at least one sensor adapted to be or being physically connected to or integrated with the time-of-flight image sensor to measure low-frequency magnetic fields generated by a transmitter source.

In an embodiment, the position tracking means comprises a MEMS sensor magnetic motion capture system adapted to be or being physically connected to or integrated with the time-of-flight image sensor to measure return signal upon activation by a transponder.

In an embodiment, the position tracking means comprises an acoustic sensor including at least one sensor mounted on or integrated with the time-of-flight image sensor for increase accuracy - e.g. for the determination of direction and/or orientation of the laparoscopic tool.

In an embodiment, the position tracking means comprises at least one distance sensor, such as a short range laser based sensor e.g. as provided by SICK AG, Germany.

A short range laser based distance sensor is operating by projecting a light beam spot onto a measurement object, e.g. using a laser diode. By means of an optical receiver, the reflection is mapped onto a light sensitive element (such as CMOS). Based on the position of the mapped light spot and the 3D surface data, the distance to the surface area may be determined.

In an embodiment, the spatial position tracking means comprises at least one local reference sensor, preferably for spatial reference. Advantageously the at least one local reference sensor is adapted to being positioned on a patient and/or on a support (surgery table) for a patient.

In an embodiment there are a plurality of reference sensors, the reference sensors are preferably configured to communicate to locate a position of each other to thereby define an X.Y.Z dimensional space - e.g. as described in US 2007/060098 or US 6,631,271. In an embodiment, the 3D surface sensor system is as described in "Spatial Data Estimation in Three Dimensional Distributed Wireless Sensor Networks", by Karjee et al. Embedded Systems (ICES), 2014 International Conference 3-4 July 2014, IEEE ISBN 978-1-4799-5025-6.

In an embodiment the laparoscopic tool forms part of or is mounted on a robot for motorized manoeuvring of the laparoscopic tool and the spatial position tracking means comprises a motion sensor wherein the motion sensor is adapted for determining motions of the laparoscopic tool at least partly based on the motorized manoeuvring of the laparoscopic tool.

The robot may be motorized by use of any suitable motor(s) or motor systems e.g. comprising one or more step motors and/or one or more actuators.

In an embodiment, the spatial position tracking means comprises at least one sensor mounted to the distal portion of the laparoscopic tool.

Advantageously the spatial position tracking means comprises at least one of a global position sensor and/or a local position sensor.

In a preferred embodiment, the spatial position tracking means is adapted for detecting changes in rotational attributes, preferably comprising pitch, roll and yaw and optionally the spatial position tracking means is adapted for detecting acceleration.

In an embodiment, the spatial position tracking means comprises an inertial measurement system, such as an inertial measurement unit (IMU), the inertial measurement system preferably comprises at least one of an accelerometer, a gyroscope and/or a magnetometer. Advantageously the spatial position tracking means comprises an IMU enabled GPS device. In this embodiment, it is desired that the spatial position tracking means is adapted for tracking the spatial position of the time-of-flight image sensor relative to a selected spatial start position as described above.

In an embodiment, the spatial position tracking means comprises a structured light projecting unit mounted to the distal portion of the laparoscopic tool and adapted for projecting a structured light pattern, wherein the structured light pattern differs in wavelength(s) from one or more wavelengths emitted by the time-of-flight light emitter.

In an embodiment, the spatial position tracking means comprises a depiction system for generating a real time correlated depiction of movements of a laparoscopic tool as described in DK PA 2015 70642.

In an embodiment, the spatial position tracking means comprises visual identifier adapted for tracking positions and orientations of the time-of-flight image sensor. Such a visual identifier is for example in the form of a pico-lantern e.g. as described by Philip Edgcumbe1 et al in "Pico Lantern: A Pickup Projector for Augmented Reality in Laparoscopic Surgery" Med Image Comput Comput Assist Interv. 2014;17(Pt 1):432-9. PMID:25333147.

In principle the time-of-flight light emitter of the laparoscopic tool system of the time-of flight camera set may be positioned anywhere provided that at least a portion of the emitted light reaches and is reflected from the surface area of the surgery cavity.

In an embodiment, the time-of-flight light emitter is adapted to be positioned at a distance to the time-of-flight image sensor, such as at a unit adapted to be held substantially immovable.

To obtain a very accurate resolution, it is desired that the time-of-flight light emitter is either held at a stationary position and/or that the position or changes of position relative to the time-of-flight image sensor and/or to a X,Y,Z coordinate matrix is/are known.

Usually the laparoscopic tool may be calibrated with the time-of-flight light emitter in one or more selected positions and the measurements are thereafter based on this or these calibrations, e.g. modified in view of the movements of the time-of-flight light emitter.

In an embodiment, the time-of-flight light emitter is adapted to be positioned at a distance to the time-of-flight image sensor. The time-of-flight light emitter may for example be positioned on a distal portion of a further laparoscopic tool, such as a laparoscope. In an embodiment a further spatial position tracking means - e.g. as the position tracking means described above - is adapted to tracking the spatial position of the time-of-flight light emitter.

Thereby the time-of-flight image sensor and the time-of-flight light emitter may be moved independently of each other while 3D real life images are generated by the laparoscopic tool system. Since the position of both the time-of-flight image sensor and the time-of-flight light emitter are known, the computer system may account for such independent movements and calculate 3D data for the surface area.

In an embodiment, the time-of-flight light emitter is adapted to be positioned closer to the surface to be analyzed than the time-of-flight image sensor. For example the time-of-flight image sensor is positioned on the distal portion of a cannula or an endoscope and the time-of-flight light emitter is positioned on a distal portion of a further laparoscopic tool in the form of a laparoscopic instrument.

Thereby the time-of-flight image sensor may be held relatively far from the surface while still highly accurate dynamic real life measurements may be obtained because of the time-of-flight light emitter, which may be relatively close to the surface area. The risk of interference and/or cross-talk between pixels/ point sensor units of the time-of-flight image sensor is reduced, and accordingly signals with reduced noise are obtained.

an embodiment, the time-of-flight camera module is mounted to or incorporated in a laparoscopic instrument and the time-of-flight image sensor and time-of-flight light emitter is positioned at the distal end of the laparoscopic instrument. This may allow both 3D surface scanning and information on instrument position directly to the operator.

The time-of-flight light emitter of the laparoscopic tool system is advantageously adapted for emitting modulated light.

The term "modulated light" means light that is timely variated in a preselected timely pattern, e.g. with a modulation frequency (or merely called frequency) which may be stationary or may vary according to a preselected variation pattern.

The modulated light may be pulsed or continuous-wave (CW) modulated light. The modulation frequency largely depends on the type of modulation. In an embodiment the modulation frequency is at least about 200 Hz, such as at least about 100 KHz, such as at least about 1 MHz, such as at least about 20 MHz, such as up to about 200 MHz or more.

To ensure a high resolution it is preferred that the modulation frequency is at least 200 Hz and advantageously at least about 1MHz.

In an embodiment the emitted light is pulsed light and the modulation is the pulsing of the light. The pulsed light need not be further modulated, however if desired it may additionally be modulated by one or more other modulation types, such as pulse duration and/or any of the below mentioned modulation types.

In an embodiment the pulsed light is additionally modulated with respect to pulse rep rate e.g. by using a pulse dumper/pulse picker to dump out pulses in a preselected pattern.

In an embodiment the emitted light is CW light and the light is modulated by modular changes of at least one of amplitude (and thereby the intensity), phase, light frequency, pulse frequency, polarization and/or pulse length of the light, preferably the time-of-flight light emitter comprises a tunable modulator.

Generation of such types of light modulations is well known to a skilled person.

For high and fast resolution it is desired that the modulation frequency is at least about 200 Hz, such as at least about 100 KHz, such as at least about 1 MHz, such as at least about 20 MHz, such as up to about 200 MHz or more.

In an embodiment the time-of-flight light emitter is adapted for emitting light at a power of from about 1 mW to about 100 W.

Preferably the time-of-flight light emitter of the laparoscopic tool system of the is emitting spatially incoherent light at a power of from about 1 W to about 50 W.

In an embodiment the time-of-flight light emitter is adapted for emitting a spatially coherent light beam and the emitter is further adapted
- for emitting a scanning beam or
- for emitting two or more stationary (non scanning) beams for triangular determinations.

Triangular determinations are well known in the art to determine spatial relations, sizes and/or other 3D related dimensions.

It is however preferred that the time-of-flight light emitter is adapted for emitting incoherent light, thereby providing a relatively large spot size onto the surface area.

Preferably, the time-of-flight light emitter of the time-of flight camera set comprises at least one light emitting diode (LED), optionally the light emitter comprises two or more equal or different LEDs.

The selected wavelength(s), band width, tenability and power may be as described above.

The time-of-flight image sensor is preferably a camera comprising an array of light sensor units, such as a photo detector e.g. a photo-electric sensor unit converting light energy (photons) into electricity (electrons).

Preferably, the camera comprises an array of pixel sensors each comprising a photodetector (such as an avalanche photodiode (APD), a photomultiplier or a metal-semiconductor-metal photodetector (MSM photodetector). Preferably the time-of-flight image sensor comprises active pixel sensors (APS), preferably each pixel comprises an amplifier, more preferably the time-of-flight image sensor comprises at least about 1 kilo pixels, such as at least about 1 Mega pixels.

Preferably, the camera is a time-of-flight camera build using MEMS technology.

The time-of-flight camera preferably comprises a demodulator for demodulating the received light of each pixel sensor to determine the real life 3D shape.

The demodulator may advantageously form part of the computer system. In an embodiment, the computer system comprises a demodulator positioned at a distance to the time-of-flight camera set.

Advantageously the time-of-flight camera comprises a band pass filter for suppressing back-ground light.

The camera preferably has a relatively short integrating time for fast operation and for high accuracy also when the camera is moved.

Preferably, the time-of-flight camera is operatively connected to the time-of-flight light emitter, preferably for timely adjusting the operation of the emitter and the receiver.

Where the emitter is adapted for emitting pulsed light the receiver isadvantageously timely synchronized with the time-of-flight light emitter.

To increase the sensitivity of the camera, the time-of-flight camera advantageously comprises at least one aperture lens, such as a Fresnel lens for collecting reflected light.

Advantageously the time-of-flight image sensor is selected from a charge-coupled device (CDD) image sensor and a complementary metal-oxide-semiconductor (CMOS) image sensor.

When using CMOS or other integrating detectors the camera preferably has a selectable integrating time, comprising settings of relatively low integration time such as 10 ms or less, such as 1 ms or less. Longer integration time may be used for calibration - e.g. up to 100 ms or longer. Also for relatively curved and hilly surface areas a longer integration time may be used.

The laparoscopic tool system May in addition comprise the time-of-flight distance sensor set The time-of-flight distance sensor set is provided as the position tracking means of the laparoscopic tool system.

Thus, in a preferred embodiment the spatial position tracking means comprises a time-of-flight distance sensor set for determining a distance between the distal portion of the laparoscopic tool and a surface area.

Preferably, the time-of-flight light emitter of the time-of-flight distance sensor set and the time-of-flight light emitter of the time-of-flight camera set are integrated with each other to a combined time-of-flight light emitter for the time-of-flight distance sensor set and the time-of-flight camera set.

In some minimally invasive surgery procedure the laparoscopic instrument is operated to burn off tissue areas which thereby generates smoke.

In an embodiment, the laparoscopic tool system further comprises an image camera secured to the laparoscopic tool at its distal portion and configured for acquiring images and transmitting the acquired image to the computer system and the computer system is configured for correcting the images based on the light data received from said time-of-flight receiver and/or time-of-flight image sensor. The image camera may be a stereo camera.

Thereby, if the minimally invasive surgery cavity is filled with smoke, fog or similar which reduces the quality of the images obtained by the image camera, the image of the image camera is corrected to remove errors occurred due to the smoke, fog or similar.

The laparoscopic tool system may comprise a time-of-flight distance sensor set specifically focused on making it simpler for the operator to manipulate a laparoscopic instrument with high precision during a laparoscopic procedure by providing the operator with real life information about a distance between a surface area and the laparoscopic instrument. The surface area is advantageously a tissue surface area within the surgery cavity where the minimal invasive surgery is to be performed, e.g. a target area for the laparoscopic procedure.

Often the surface of the minimally invasive surgery cavity is very curved. The term 'target area' of the surface of the minimally invasive surgery cavity is herein used to designate the area which the operator has focus on, and the target area may advantageously comprise a surgery site and/or a tissue surface area which potentially could be in risk of damage during the laparoscopic procedure.

In an embodiment, the time-of-flight light emitter and the time-of-flight receiver of the time-of-flight distance sensor set are fixed to the distal portion of the laparoscopic tool, the time-of-flight light emitter is adapted to emitting light and the time-of-flight receiver is adapted to sensing reflected light emitted by the time-of-flight emitter and to generating received light data when reflected from a surface area within a preselected distance from the time-of-flight distance sensor set.

The time-of-flight light emitter and the time-of-flight receiver time-of-flight distance sensor set are preferably rigidly fixed to the distal portion of the laparoscopic tool to ensure high accuracy. The time-of-flight light emitter and the time-of-flight receiver time-of-flight distance sensor set may in an embodiment have two or more settings of emitting/receiving directions. Such settings are preferably step-wise for simpler calibration of the time-of-flight distance sensor set.

In that way the operator may receive distance information from the laparoscopic instrument to different parts of a selected surface area within the surgery cavity.

The optional plurality of settings of the emitting/receiving directions is advantageously provided by an angular tilting movement in one or more directions by a step motor, which may be operated by a suitable controller arranged at a handle portion of the laparoscopic instrument or operated by a robotic operator.

The term "emitting direction" means the optical axis of the emitted light and receiving direction" means the optical axis of the received (collected) light.

The computer system is advantageously in data communication with the time-of-flight distance sensor set to receiving the received light data.

It should be understood herein that the term "in data communication" may include any type of data communication capable of transmitting/receiving digital or analog data signal. For simplification it is generally desired to use digital data communication or wire based communication. The data communication may thus be by wire or wireless, e.g. using Bluetooth or similar data transmission systems.

Where the operator is a robot it may be desired to use wire based communication and the computer system or at least a part thereof may advantageously be integrated with the robot.

The computer system may be programmed to calculate a real time distance between at least a part of the distal portion and the surface area.

Thus since the operator may be constantly informed about the distance between the laparoscopic instrument and the surface area - e.g. one or more points of the surface area - the risk of unintended damaging of tissue is significantly reduced and the operator may be able to perform the laparoscopic procedure with less movements of the laparoscopic tool and/or substantially faster than without the laparoscopic tool system comprising the time-of-flight distance sensor set.

In an embodiment, the laparoscopic instrument is a dynamic instrument configured for performing a laparoscopic surgery procedure on the surface area within the minimally invasive surgery cavity. The laparoscopic instrument is adapted for being moved both in axial direction relative to the laparoscopic instrument but also in several tilting directions for performing a minimally invasive surgery procedure.

A laparoscopic surgery procedure means herein a minimally invasive surgery procedure where the laparoscopic instrument is inserted into via a previously formed access port into a minimally invasive surgery cavity to be in interaction with tissue within the minimally invasive surgery cavity by or in the vicinity of the tissue surface area to perform a surgical action on the tissue. A laparoscopic examination procedure Is a minimally invasive surgery procedure where the tissue within the minimally invasive surgery cavity is examined for diagnostic purpose, e.g. for planning and/or for providing information to the operator if and/or how a laparoscopic surgery procedure should be performed.

The time-of-flight light emitter and the time-of-flight receiver of the time-of-flight distance sensor set are advantageously configured to be correlated to each other to ensure that the distance, angle and other position based parameters are adjusted to ensure low measurement error.

In an embodiment, the time-of-flight light emitter and the time-of-flight receiver of the time-of-flight distance sensor set are integrated into a time-of-flight distance module. Thereby the time-of-flight light emitter and the time-of-flight receiver are spatially correlated. Advantageously the time-of-flight light emitter and the time-of-flight receiver are rigidly interconnected such that an optional movement from a first setting of the emitting/receiving directions to another will be an equal movement of both of the time-of-flight light emitter and the time-of-flight receiver, thereby providing a simpler calibration.

In an embodiment the time-of-flight distance sensor set is not a rangefinder.

In an embodiment, the time-of-flight distance module comprises a rangefinder.

Advantageously the time-of-flight distance sensor set is configured for dynamic operation during the minimally invasive surgery procedure while the laparoscopic instrument is dynamically moved with movements including tilting in various directions, such that the operator receives real-time information about the distance between the distal portion of the laparoscopic instrument and the tissue within the minimally invasive surgery cavity.

In an embodiment, the time-of-flight distance module is preferably a rangefinder, preferably in miniature format. Rangefinders are well known within the art of hunting and are used to determine the distance to a target animal. The principle used in such range finders may be applied in the time-of-flight distance module, preferably modified to measure distances of up to 1 m such as up to 0.5 m, e.g. from about 1 mm to about 25 cm. In an embodiment, the time-of-flight distance module is a miniature distance sensors as marketed by Sensopart, Germany. In an embodiment the time-of-flight distance module comprises at least one distance sensor, such as a short range laser based sensor e.g. as provided by SICK AG, Germany.

Advantageously the computer system comprises at least one computer unit, which is integrated with the time-of-flight receiver and/ or the time-of-flight light emitter. The incorporated computer unit may for example be applied to control the operation of the time-of-flight receiver and/ or the time-of-flight light emitter and to ensure an operative correlation between the time-of-flight receiver and the time-of-flight light emitter.

The time-of-flight light emitter of the time-of-flight distance sensor set may be coherent, partially coherent or non-coherent.

Coherence is strongly related to the ability of light to exhibit interference effects. A light field is called coherent when there is a fixed phase relationship between the electric field values at different locations or at different times. Partial coherence means that there is some (although not perfect) correlation between phase values. There are various ways of quantifying the degree of coherence, as described below.

There are two very different aspects of coherence:
Spatial coherence means a strong correlation (fixed phase relationship) between the electric fields at different locations across the beam profile. For example, within a cross-section of a beam from a laser with diffraction-limited beam quality, the electric fields at different positions oscillate in a totally correlated way, even if the temporal structure is complicated by a superposition of different frequency components. Spatial coherence is the essential prerequisite of the strong directionality of laser beams.

Temporal coherence means a strong correlation between the electric fields at one location but at different times. For example, the output of a single-frequency laser may exhibit a very high temporal coherence, as the electric field temporally evolves in a highly predictable fashion: it exhibits a clean sinusoidal oscillation over extended periods of time.

Advantageously the time-of-flight light emitter of the time-of-flight distance sensor set is adapted for emitting a spatially and/or temporally coherent light beam. Thereby it is simpler to measure the distance to a selected surface area such as a point at the surface area in particular where the surface area is relatively uneven and undulating.

In an embodiment the emitter of the time-of-flight distance sensor set preferably comprises a laser, such as a semiconductor laser (sometimes called a semiconductor laser diode or merely a laser diode LD).

Whereas it for distance measurement is desired to use spatially coherent light, spatially incoherent light may in an embodiment be applied. This embodiment is in particular suitable where the surface area is relatively even.

In an embodiment, the time-of-flight light emitter of the time-of-flight distance sensor set comprises at least one of a laser or a light emitting diode (LED), optionally the light emitter comprises two or more equal or different lasers and/or LEDs, such as two or more semiconductor lasers.

Generally, the semiconductor LEDs on the market today emit incoherent light. The incoherent light is for example generated by spontaneous emission which may provide the semiconductor LEDs to produce light waves that lack a fixed-phase relationship. LEDs are often preferred due to their relatively low cost.

Semiconductor laser diodes (LD) which are preferred for the emitter of the time-of-flight distance sensor set, preferably emits spatially and temporally coherent light.

Generally the LDs on the market today produce light waves with a fixed-phase relationship (both spatial and temporal) between points on the electromagnetic wave. Light waves having a fixed-phase relationship are referred to as temporally coherent light. Since semiconductor LDs emit more focused light than LEDs, they are preferred for use in the determination of the distance between at least a part of the distal portion and the surface area in particular where the surface area is very undulating and irregular. In an embodiment, the time-of-flight light emitter of the time-of-flight distance sensor set comprises a vertical-cavity surface-emitting laser (VCSEL).

The time-of-flight light emitter is advantageously adapted for emitting a light with a relatively narrow bandwidth. Thereby it is simpler to control and/or adjust for optional penetration of certain wavelengths and also the risk of interference, cross-talk and/or noise due to other light beams may be held at a very low level.

In an embodiment, the time-of-flight light emitter of the time-of-flight distance sensor set comprises a light source having a band width (full width at half maximum -FWHM) of up to about 50 nm, such as from about 0.5 nm to about 40 nm such as from about 1 to about 10 nm.

The time-of-flight light emitter of the time-of-flight distance sensor set may in principle be configured for emitting any wavelength(s). The emitting wavelength(s) is/are advantageously selected in dependence of optional other light in the surgery cavity (i.e. to be distinguishable from such other light), in dependence of cost, in dependence of optional heat generation (i.e. to avoid undesired heating) and/or in dependence of penetration and/or absorptions properties (i.e. to ensure a highly reliable reflection of the emitted light at the surface area).

In an embodiment the time-of-flight light emitter of the time-of-flight distance sensor set is advantageously adapted for emitting at least one electromagnetic wavelength within the UV range of from about 10 nm to about 400 nm, such as from about 200 to about 400 nm.

In an embodiment the time-of-flight light emitter of the time-of-flight distance sensor set is adapted for emitting at least one electromagnetic wavelength within the visible range of from about 400 nm to about 700 nm, such as from about 500 to about 600 nm.

In an embodiment the time-of-flight light emitter of the time-of-flight distance sensor set is adapted for emitting at least one electromagnetic wavelength within the IR range of from about 700 nm to about 1mm, such as from about 800 to about 2500 nm.

The time-of-flight light emitter of the time-of-flight distance sensor set may advantageously comprise a wavelength tunable light source. The wavelength tunable light source may advantageously be tunable by the operator e.g. to switch between preselected wavelengths or wavelengths ranges.

In an embodiment, the time-of-flight light emitter of the time-of-flight distance sensor set comprises two or more light sources having equal or different bandwidths which preferably have different penetration depth and/or are different with respect to absorption of at least one type of tissue, blood and/or water. The two or more pattern light sources are preferably operatively interconnected to ensure that there is no interference or cross-talk between them. In an embodiment, the two or more pattern light sources are operatively interconnected to emit light in an alternating order.

The power of the time-of-flight light emitter may advantageously be selectable (tunable) by the operator to be selected for the respective laparoscopic procedure.

In an embodiment, the time-of-flight light emitter of the time-of-flight distance sensor set is adapted for emitting light at a power of from about 1 mW to about 100 mW, such as from about 3 mW to about 50 mW.

Where the time-of-flight light emitter of the time-of-flight distance sensor set is emitting spatially coherent light the time-of-flight light emitter is preferably adapted for emitting light at a relatively low power e.g. less than 100 mW, whereas where the time-of-flight light emitter of the time-of-flight distance sensor set is emitting spatially incoherent light the time-of-flight light emitter may be arranged for emitting light at a relatively high power even up to about 100 W, such as up to about 50 W, such as up to 10 W, such as up to about 5 W.

The time-of-flight receiver may be any receiver capable of receiving and detecting light and demodulating the received light. Advantageously the time-of-flight receiver comprises a photodetector, such as an avalanche photodiode (APD), a photomultipliers or a metal-semiconductor-metal photodetector (MSM photodetector).

Such receivers are known from Rangefinders.

The time-of-flight receiver preferably comprises a demodulator for demodulating the received light to determine the time it has taken the light to pass from the time-of-flight light emitter to the time-of-flight receiver and thereby determine the distance between the laparoscopic instrument and the surface area where the light was reflected. The demodulator may advantageously form part of the computer system. In an embodiment the computer system comprises a demodulator positioned at a distance to the time-of-flight distance sensor set.

Advantageously the time-of-flight receiver comprises a band pass filter for suppressing back-ground light.

Preferably, the time-of-flight receiver is operatively connected to the time-of-flight light emitter of the time-of-flight distance sensor set, preferably for timely adjusting the operation of the emitter and the receiver.

Where the emitter is adapted for emitting pulsed light the receiver is advantageously timely synchronized with the time-of-flight light emitter of the time-of-flight distance sensor set.

To increase the sensitivity of the receiver, the time-of-flight receiver advantageously comprises at least one aperture lens, such as a Fresnel lens for collecting reflected light.

All features of the inventions and embodiments of the invention as described herein including ranges and preferred ranges may be combined in various ways within the scope of the invention, unless there are specific reasons not to combine such features.

### Brief description of preferred embodiments and elements of the invention

The above and/or additional objects, features and advantages of the present invention will be further elucidated by the following illustrative and nonlimiting description of embodiments of the present invention, with reference to the appended drawings.
Fig. 1 is a schematic side view illustration of a laparoscopic tool.
Fig. 2 is a perspective view of a minimally invasive surgery procedure seen from the outside of a patient.
Fig. 3 is a schematic illustration of a cannula comprising a time-of-flight light emitter.
Fig. 4 is a schematic illustration of a variation of the cannula of Fig. 3.
Fig. 5 is a schematic illustration of a laparoscopic tool system comprising a time-of-flight distance sensor set.
Fig. 6 is a schematic illustration of a laparoscopic tool system comprising a time-of-flight distance sensor set.
Fig. 7 is a schematic illustration of a further laparoscopic tool system comprising a time-of-flight distance sensor set.
Figs. 8a and 8b are illustrations of time-of-flight receivers suitable for the laparoscopic tool system comprising a time-of-flight distance sensor set.
Fig. 9 is a schematic illustration of a laparoscopic instrument comprising a time-of-flight distance sensor set.
Figs. 10a-10d illustrate coherent and incoherent light.
Figs. 11a-11d illustrate examples of modulation of light.
Fig. 12 is a schematic illustration of an embodiment of a laparoscopic tool system comprising a time-of-flight camera set.
Fig. 13 is a schematic illustration of another embodiment of a laparoscopic tool system comprising a time-of-flight camera set.
Fig. 14 is a schematic illustration of an embodiment of a laparoscopic tool system comprising a time-of-flight camera set.
Figs. 15a and 15b are illustrations of time-of-flight cameras suitable for the laparoscopic tool system comprising a time-of-flight camera set.
Fig. 16 is a schematic illustration of a XYZ coordinate matrix and further illustrates preferred functions of the position tracking means.
Fig. 17 is a schematic illustration of an embodiment of a laparoscopic tool comprising a spatial position tracking means, which is adapted for detecting changes in rotational attributes, preferably comprising pitch, roll and yaw.
Fig. 18 is a perspective view of a minimally invasive surgery procedure seen from within the cavity.
Fig. 19 is a perspective view of a minimally invasive surgery procedure seen from within the cavity and performed using a laparoscopic tool system of an embodiment of the invention.
Figs. 20a and 20b are examples of distal portions of laparoscopic tools comprising a time-of-flight camera set or a time-of-flight distance sensor set.
Fig. 21 illustrates a laparoscopic tool comprising a time-of-flight camera set during a MIS procedure.
Fig. 22 illustrates a laparoscopic tool comprising a time-of-flight distance sensor set during a MIS procedure.
Fig. 23 illustrates another laparoscopic tool comprising a time-of-flight camera set during a MIS procedure.
Fig. 24 illustrates another laparoscopic tool comprising a time-of-flight distance sensor set during a MIS procedure.
Fig. 25 illustrates a further laparoscopic tool comprising a time-of-flight camera set during a MIS procedure.

The figures are schematic and may be simplified for clarity. Throughout the same reference numerals are used for identical or corresponding parts.

Further scope of applicability of the present invention will become apparent from the description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

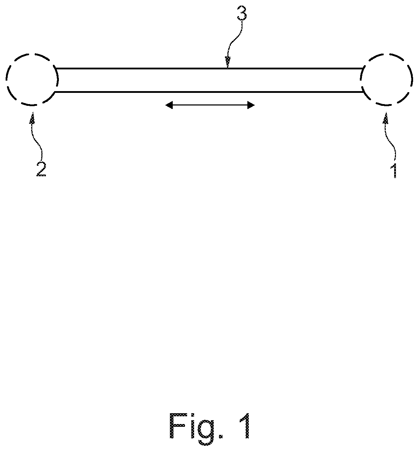

The laparoscopic tool of Fig. 1 comprises a proximal portion 2 and a distal portion 1. The proximal portion 2 and a distal portion 1 are interconnected by a shaft 3 which is advantageously rigid. The shaft may in an embodiment be flexible and/or comprise one or more bendable and/or pivotable joints. Preferably such flexibility and or joints are controlled by the operator. The distal portion 1 may be a surgical tool or the distal part of a cannula or a laparoscope. The distal portion 1 and a part of the shaft 3 are adapted to being within the surgery cavity during a surgical procedure. The proximal portion 2 is adapted to remain outside the surgery cavity and is for example a handle portion for maneuvering the laparoscopic tool. In an embodiment, the proximal portion is a collar such as a collar of a cannula. In an embodiment the proximal portion is a connection to a robot for maneuvering the laparoscopic tool. The proximal portion may comprise various control means, such as on/off button(s) for switching light and camera/receiver and optionally other elements on and off as well as features for controlling and operating an optional instrument and/or optional joints of the shaft.

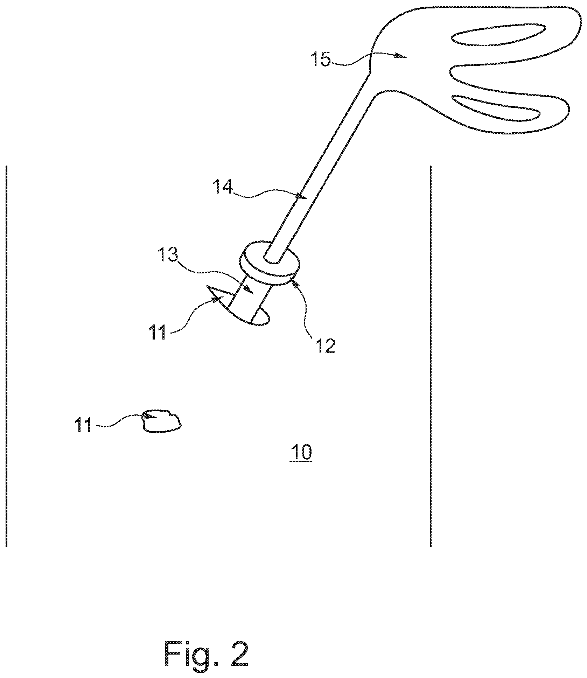

Fig. 2 shows the outer skin 10 of a patient during a minimally invasive surgery procedure. Two incisions are made through the skin 10 of the patient and in a first of the incisions a cannula with a shaft 13 and a collar 12 are inserted. A not shown distal portion of a laparoscopic tool with a shaft 14 and a handle 15 are inserted through the access port of the cannula.

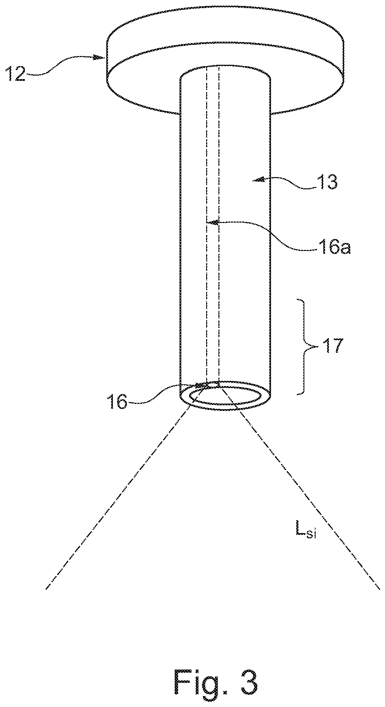

The cannula shown in Fig. 3 corresponds or is identical to the cannula shown in Fig. 2 and comprises the shaft 13 and the collar 12. At the distal portion 17 of the cannula it comprises a time-of-flight light emitter 16 - which in the shown embodiment is illustrated to emit spatially incoherent light Lₛᵢ. Via a channel the light may be transmitted via a not shown optical fiber.

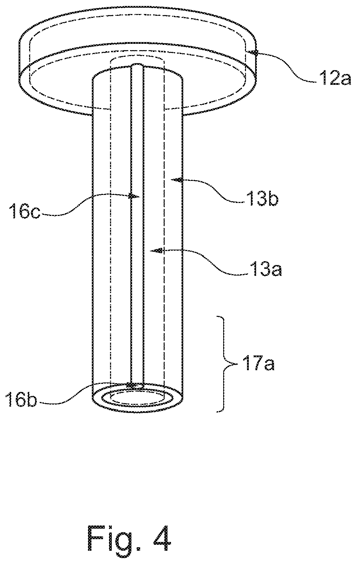

The cannula shown in Fig. 4 is a variation of the cannula of Fig. 3 and comprises a shaft 13a and the collar 12a. A sleeve 13b is mounted onto the shaft and distal portion 17a. A time-of-flight light emitter 16b is mounted onto the distal portion 17a via the sleeve 13b. A not shown optical fiber is arranged along the sleeve 17b and guides the light to the emitter 16b. The cannula may advantageously also comprise a time-of-flight camera and/or a time-of-flight receiver.

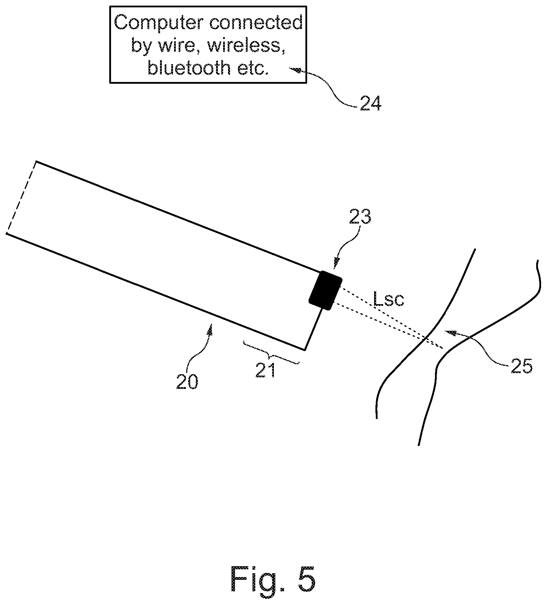

Fig. 5 show a laparoscopic tool system comprising a laparoscopic instrument 20, a time-of-flight distance sensor set in the form of a time-of-flight distance sensor module 23 and a computer system 24.

The laparoscopic instrument 20 comprises a distal portion 21 with a not shown surgical tool. The time-of-flight distance sensor module 23 is fixed at the distal portion 21 of the laparoscopic instrument 20. The time-of-flight distance sensor module 23 comprises a time-of-flight light emitter and a time-of-flight receiver. The time-of-flight light emitter emits light L_{sc} that at least is spatially coherent. The emitted light may advantageously also be temporally coherent. The light L_{sc} is impinging on the surface area 25 and at least a part of the light is reflected by the surface area 25 and captured by the time-of-flight receiver of the time-of-flight distance sensor set 23. By determining the time it takes the emitted light to reach the time-of-flight receiver, the distance between the distal portion 21 of the laparoscopic instrument 20 and the surface area 25 may be determined. The computer system 24 is schematically illustrated and may comprise two or more separate computing elements in data connection with each other - e.g. one or more drivers, one or more modulators and demodulators, one or more data storing elements, such as one or more data bases, etc. The various elements of the computer system are adapted to be at least temporarily in data connection with each other for transferring the data required to operate the time-of-flight distance sensor set and performing the distance determination. In an embodiment one or more elements of the computer system is/are integrated in the time-of-flight distance sensor set, e.g. one or more drivers and/or one or more modulators and/or demodulators. The data transmission may be by wire or be wireless, e.g. using blue tooth.

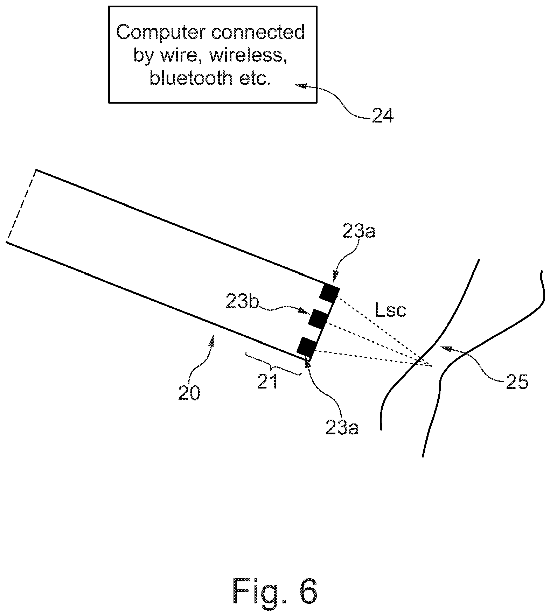

Fig. 6 shows another embodiment of a laparoscopic tool system. The laparoscopic tool of Fig. 6 differs from the laparoscopic tool system of Fig. 5 in that the time-of-flight distance sensor set is not in the form of a module but here it comprises one time-of-flight light emitter 23b and two time-of-flight receivers 23a positioned at the distal portion 21 of the laparoscopic instrument 20 at a distance from each other.

By having two or more time-of-flight receivers 23a positioned at a distance from each other at the distal portion 21 of the laparoscopic instrument 20 of the laparoscopic tool system, the system may also determine an angular position of the laparoscopic instrument relative to the surface area 25.

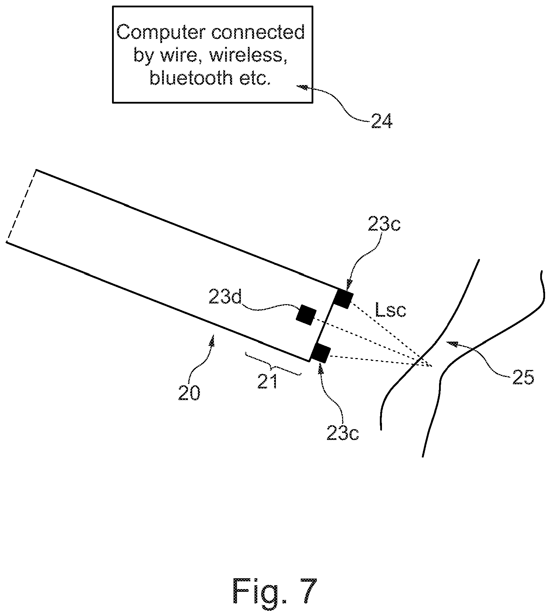

Fig. 7 shows a further embodiment of a laparoscopic tool system. The laparoscopic tool of Fig. 7 differs from the laparoscopic tool system of Fig. 5 in that the time-of-flight distance sensor set is not in the form of a module but here it comprises two time-of-flight light emitters 23c and one time-of-flight receiver 23d positioned at the distal portion 21 of the laparoscopic instrument 20 at a distance from each other. The two time-of-flight light emitters 23c are positioned at a distance to each other at the outermost end of the distal portion 21 of the laparoscopic instrument 20. Thereby any risk of the laparoscopic instrument 20 blocking the emitted light L_{sc} is very low. The light emitted by the respective time-of-flight light emitters 23c preferably differs from each other e.g. by modulation such that the receiver 23d is capable of distinguishing between the reflected and received light. Hereby the laparoscopic instrument 20 of the laparoscopic tool system may also determine an angular position of the laparoscopic instrument 20 relative to the surface area 25.

The time-of-flight receiver 23d is positioned at a distance from the outermost end of the distal portion 21 of the laparoscopic instrument 20, i.e. slightly retracted relative to the time-of-flight light emitters 23c. Thereby the risk of wetting the time-of-flight receiver 23d with blocking or blurring liquids such a blood and/or wound fluid is reduced.

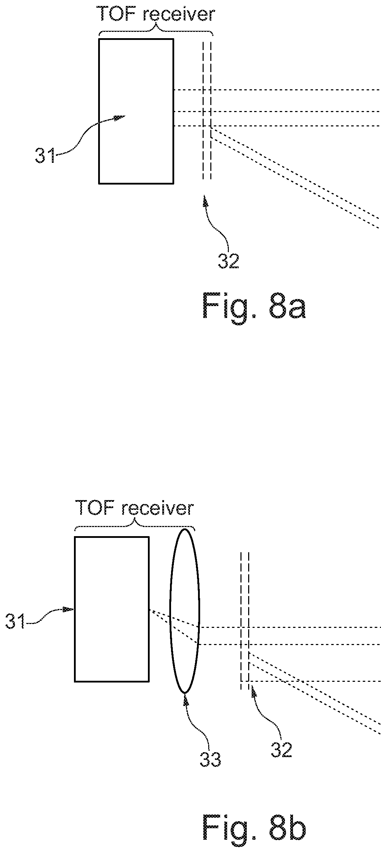

Fig. 8a shows a time-of-flight receiver comprising a photodetector module 31 optionally comprising a driver and/or a demodulator and a transmitter for wireless transmission to a not shown element of the computer system. The time-of-flight receiver also comprises a pass band filter 32, which only allows selected wavelength(s) to pass to the photodetector module 31. The pass band filter 32 is advantageously tunable: The time-of-flight receiver may also comprise a polarizing filter only allowing light of one polarizing to pass. Further, the numerical aperture, NA, of the time-of-flight receiver may be selected to receive only light propagating within a selected range of angles.

Fig. 8b shows another time-of-flight receiver which differs from the time-of-flight receiver of Fig. 8a in that it further comprises a lens 33 for collecting light. It should be understood that the lens may be replaced by a lens system comprising several optical elements including at least one lens. Preferably, such lens system is tunable.

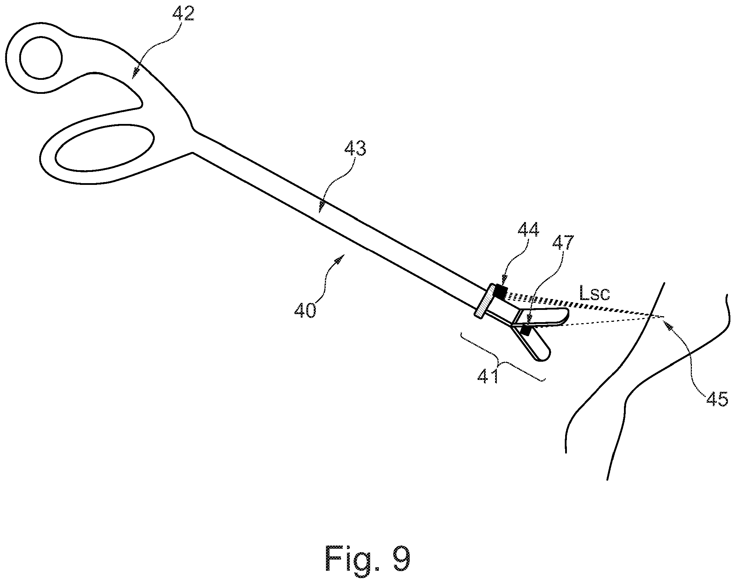

Fig. 9 shows a laparoscopic instrument 40 comprising a proximal portion 42 and a distal portion 41 comprising a surgical tool. The proximal portion 42 and a distal portion 41 are interconnected by a rigid shaft 43. A time-of-flight light emitter 44 is positioned at the distal portion 41 for emitting light L_{sc} towards a surface area 45. A time-of-flight receiver 47 is fixed at the surgical tool to receive light reflected from the surface area 45. The time-of-flight light emitter 44 and the time-of-flight receiver 47 are fixed at a known distance to each other. In a variation thereof two or more time-of-flight receivers are mounted to the distal portion 41. In an embodiment the two or more time-of-flight receivers form part of a time-of-flight camera.

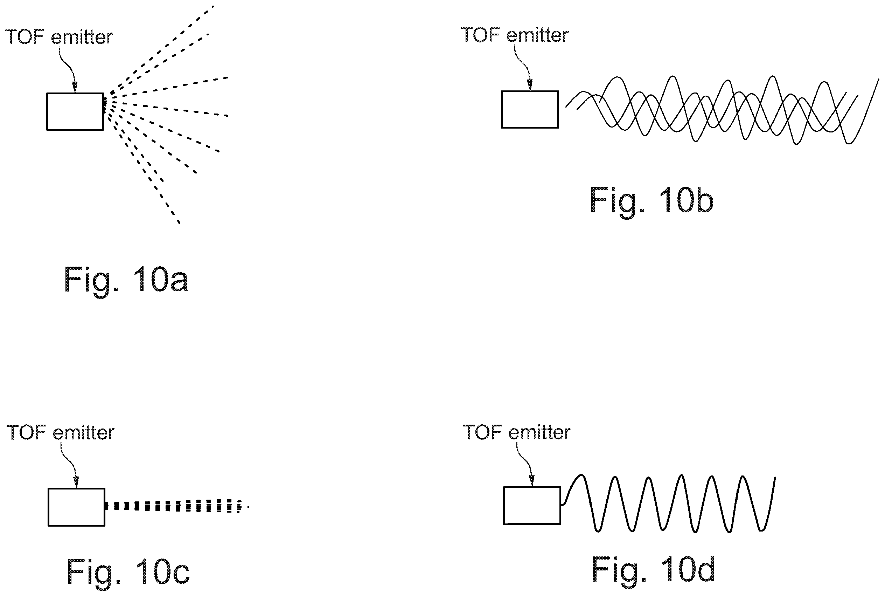

Figs. 10 illustrates a time-of-flight light emitter that emits spatially incoherent light Lsi.

Spatially incoherent light will spread out as it propagates. For time-of-flight distance sensor set, it is generally desired to use spatially incoherent light because it spreads out and covers a relatively large surface area whereas a spatially coherent light generally will have a relatively narrow spot size.

Fig. 10b illustrates a time-of-flight light emitter that emits temporally incoherent light Lₜᵢ.

Temporal coherence describes the correlation between waves observed at different moments in time. Monochromatic light is usually temporally coherent whereas broad band light is temporally incoherent.

Fig. 10c illustrates a time-of-flight light emitter that emits spatially coherent light L_{sc}.

Spatially coherent light is usually laser light.,

Fig. 10d illustrates a time-of-flight light emitter that emits temporally coherent light Ltc.

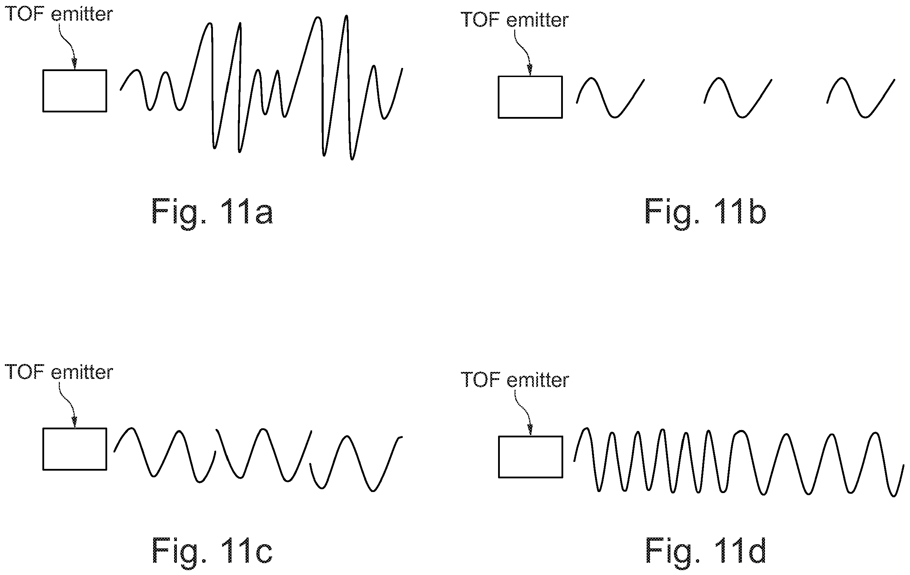

Fig. 11a illustrates amplitude modulated light. The amplitude (and thus the intensity) of the light may vary in modulated blocks of two or more intensity levels.

Fig. 11b illustrates pulsed light. The pulse frequency and/or the pulse duration may additionally be modulated.

Fig. 11c illustrates phase modulated light.

Fig. 11d illustrates wavelength modulated light.

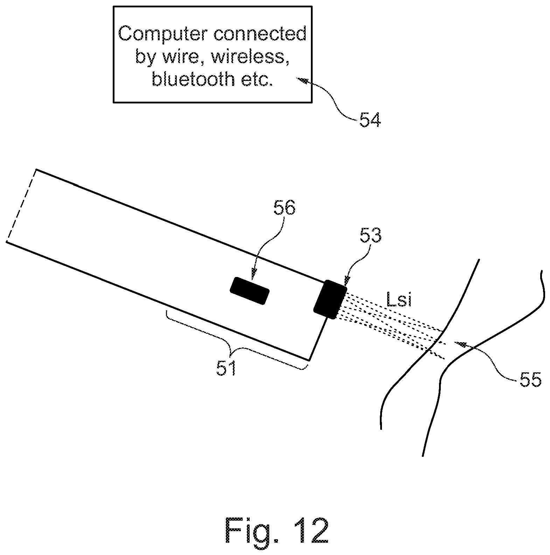

The laparoscopic tool system of an embodiment as shown in Fig. 12 comprises a laparoscopic tool 50, a position tracking means 56, a time-of-flight camera set in the form of a time-of-flight camera module 53 and a computer system 54.

The laparoscopic tool 50 comprises a distal portion 51 optionally with a not shown surgical tool. The time-of-flight camera module 53 is fixed at the distal portion 21 of the laparoscopic tool 50. The time-of-flight camera module 53 comprises a time-of-flight light emitter and a time-of-flight image sensor in the form of a time-of-flight camera. The time-of-flight light emitter emits light Lₛᵢ that is spatially incoherent. The emitted light may advantageously be temporally coherent (monochromatic). The light Lₛᵢ is impinging on the surface area 55 and at least a part of the light is reflected by the surface area 55 and captured by the time-of-flight camera of the time-of-flight camera set 53.

The time-of-flight camera comprises an array of pixels for receiving the reflected light and based on the received pattern of light and on real time spatial position data obtained from said position tracking means 56, a real time 3D image may be determined.

The spatial position tracking means 56 may be as described above. The spatial position tracking means is positioned at a known distance to the time-of-flight camera module 53.

The computer system 54 is schematically illustrated and may comprise two or more separate computing elements in data connection with each other - e.g. one or more drivers, one or more modulators and demodulators, one or more data storing elements, such as one or more data bases, etc. The various elements of the computer system are adapted to be at least temporarily in data connection with each other for transferring the data required to operate the time-of-flight camera set and performing the 3D determination. In an embodiment one or more elements of the computer system is integrated in the time-of-flight camera set e.g. one or more driver and/or one or more modulators and/or demodulators. The data transmission may be by wire or be wireless, e.g. using blue tooth.

The computer system 54 is in data communication with the spatial position tracking means 56 for receiving real time spatial position data of the time-of-flight camera module 53. Since the relative position between the spatial position tracking means and the time-of-flight camera module 53 is known the spatial position tracking means 56 may track the real time position of the time-of-flight camera module. And thus the position from where the light is emitted and received is known and the 3D determination may be performed.

The computer system 54 is also in data communication with the time-of-flight camera module 53 to receive sensed light data timely corresponding to said real time spatial position data, and the computer system is programmed to calculate the real time 3D images based on the sensed light data and the real time spatial position data.

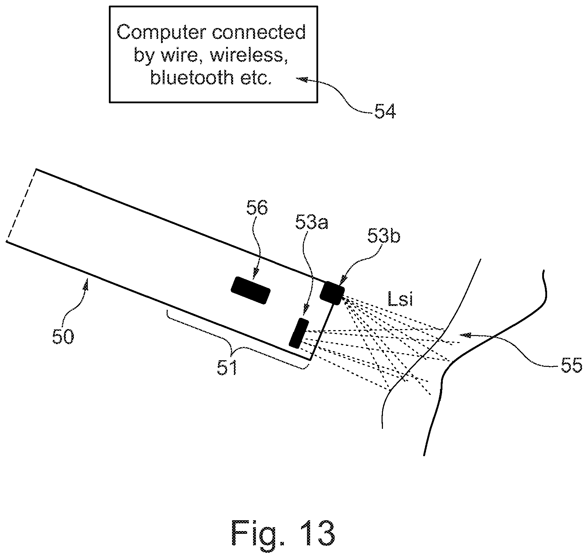

The laparoscopic tool system shown in Fig. 13 differs from the laparoscopic tool system of Fig. 12 in that the time-of-flight camera set is not in the form of a module but here it comprises one time-of-flight light emitter 53b and a time-of-flight image sensor in the form of a camera 53a, positioned at the distal portion 51 of the laparoscopic instrument 50 at a distance from each other.

By having the time-of-flight light emitter 53b and the time-of-flight image sensor 53a positioned at a distance to each other such that their relative positions are known, the system becomes more flexible and it is simpler to integrate the time-of-flight camera set into the laparoscopic tool 50.

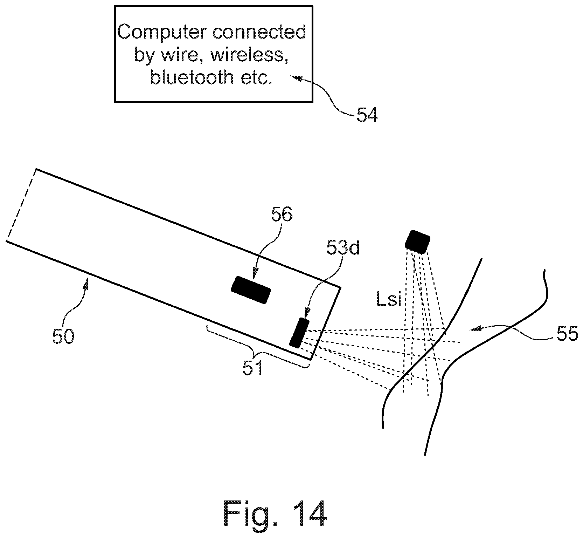

Fig. 14 shows a further embodiment of a laparoscopic tool system. The laparoscopic tool of Fig. 14 differs from the laparoscopic tool system of Fig.12 in that the time-of-flight camera set is not in the form of a module. Here it comprises one time-of-flight light emitter 53c positioned at a not shown further laparoscopic tool and a time-of-flight image sensor in the form of a camera 53d positioned at the distal portion 51 of the laparoscopic instrument 50. To know the relative positions of the time-of-flight light emitter 53c and the time-of-flight image sensor 53d, the further laparoscopic tool also comprises a not shown spatial position tracking means.

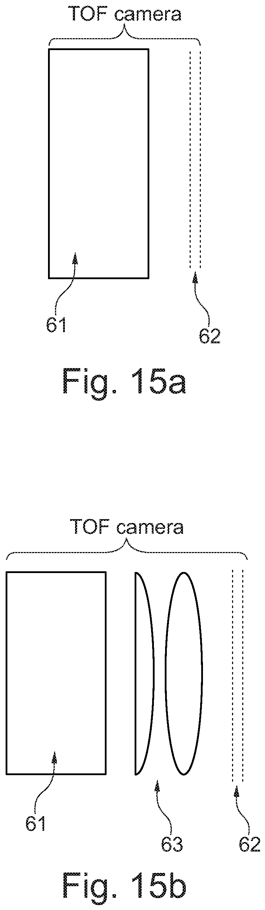

Fig. 15a shows a time-of-flight camera comprising a photodetector module 61 comprising an array of pixel sensors and optionally comprising a driver and/or a one or more demodulators and a transmitter for wireless transmission to a not shown element of the computer system. The time-of-flight camera also comprises a pass band filter 62, which only allows selected wavelength(s) to pass to the photodetector module 61. The pass band filter 62 is advantageously tunable.

Fig. 15b shows another time-of-flight image sensor which differs from the time-of-flight image sensor of Fig. 15a in that it further comprises a lens array 63 for collecting light.

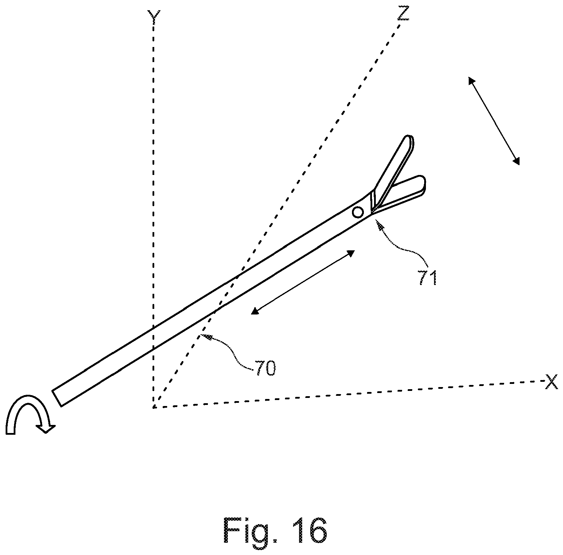

Fig. 16 illustrates a XYZ coordinate matrix for a spatial position tracking means. The spatial position tracking means determines the spatial position of the time-of-flight image sensor 71 on the laparoscopic tool relative to the XYZ coordinate matrix. The arrows indicate the movements of the laparoscopic tool 70 that are tracked by the spatial position tracking means.

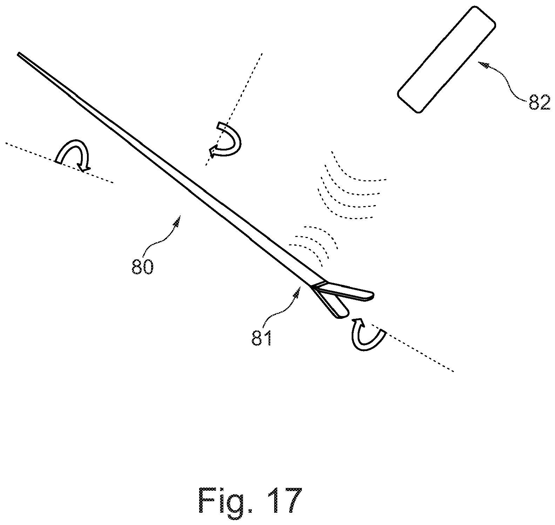

Fig. 17 shows a laparoscopic tool 80 comprising a spatial position tracking means 81, which is adapted for detecting changes in rotational attributes, preferably comprising pitch, roll and yaw. The rotational attributes are indicated by the arrows. The spatial position tracking means 81 is in wireless data communication with a computer element 82 of the computer system.

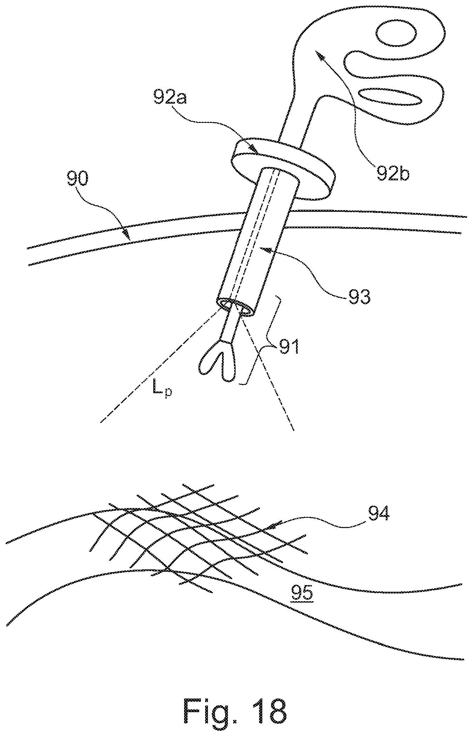

Fig. 18 shows a surgery cavity comprising a target surface area 95 below the skin 90 of a patient. A distal portion 91 of a laparoscopic instrument is inserted into the surgery cavity. The laparoscopic instrument comprises a collar 92a and a handle 92b outside the cavity. A pattern generating projector is mounted to the laparoscopic instrument by a sleeve 93 to emit a structured light beam which when impinging onto the target surface area 95 forms a pattern 94. By the size and the form of the pattern, the distance and position of the laparoscopic tool relative to the target surface area may be determined. The pattern may be monitored by a not shown camera and the obtained images may be transmitted to a not shown computer system. The pattern generation means may thus provide the spatial position tracking means of a laparoscopic tool of an embodiment of the invention. The laparoscopic tool may thus further comprise a time-of-flight camera set as described above.

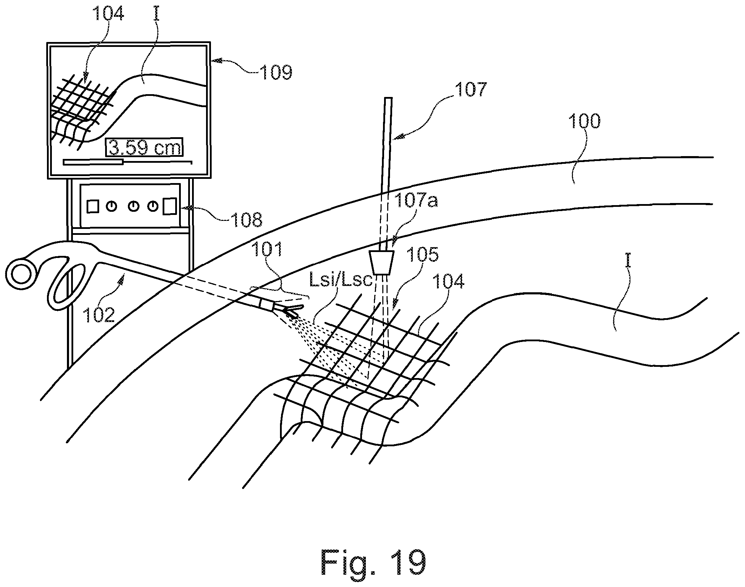

Fig. 19 shows a surgery cavity comprising a target surface area 105 e.g. comprising a part of an intestine I below the skin 100 of a patient. A distal portion 101 of a laparoscopic instrument is inserted into the surgery cavity. The laparoscopic instrument comprises a handle 102 outside the cavity for maneuvering the laparoscopic tool. The laparoscopic instrument comprises a spatial position tracking means in the form of a pattern generating projector mounted onto the laparoscopic instrument to emit a structured light beam which when impinging onto the target surface area forms a pattern 104. The laparoscopic tool further comprises a time-of-flight image sensor mounted at its distal portion 101. A further laparoscopic tool 107 is inserted into the cavity. The further laparoscopic tool 107 comprises at its distal portion a time-of-flight light emitter 107a emitting spatially coherent or incoherent light towards the target surface area 105. The light emitted by the time-of-flight light emitter 107a has a different wavelength e.g. within the invisible area than the light beam forming the pattern 104. The time-of-flight image sensor detects the reflected light from the time-of-flight light emitter 107a and advantageously also the light pattern. The time-of-flight image sensor transmits the data of the sensed light to a computer system 108, which is connected to a monitor 109. The computer calculates the distance between the laparoscopic tool and the target surface area 105 and shows it on the monitor. Advantageously also real time 3D images are determined and shown on the monitor 109. Optionally the operator may switch between 2D images and 3D images on the monitor.

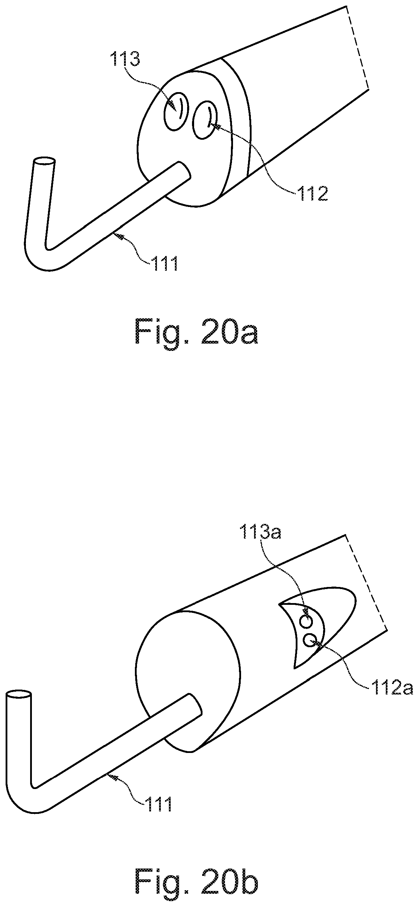

Fig. 20a shows a distal portion of laparoscopic tool. The laparoscopic tool comprises a surgical tool 111 and a time-of-flight light emitter 112 and a time-of-flight receiver 113.

Fig. 20b shows a distal portion of another laparoscopic tool that varies slightly from the one shown in Fig. 20a. The laparoscopic tool comprises a surgical tool 111 and a time-of-flight light emitter 112a and a time-of-flight receiver 113a positioned a further distance from the surgical tool 111, but still at the distal portion.

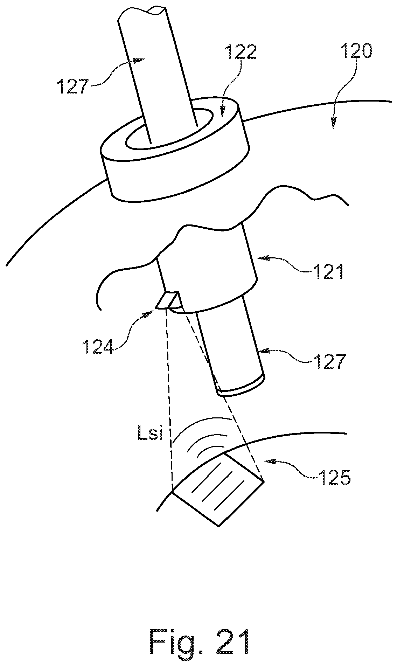

Fig. 21 shows a cannula with a distal portion 121 and a proximal portion 122 comprising a collar. The cannula is inserted through an incision in the skin 120 of a patient. The distal portion 121 of the cannula comprises a time-of-flight camera set 124 with a not shown integrated spatial position tracking means. The time-of-flight camera set emits light towards a surface area 125 and light is reflected back to the time-of-flight camera set. A laparoscopic instrument 127 is inserted through the access port of the cannula. When the laparoscopic instrument 127 is moved during the MIS procedure, the distal portion 121 of the cannula will be moved accordingly. Due to the spatial position tracking means integrated into the time-of-flight camera set, the time-of-flight camera set or the associated computer system may compensate for such movements for the 3D determination of the surface area 125.

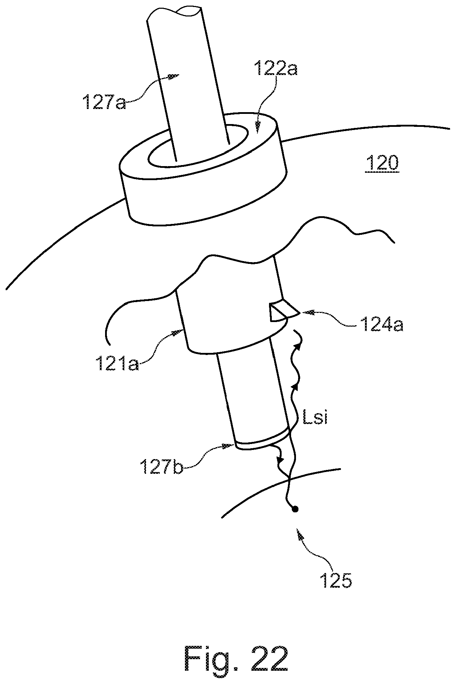

Fig. 22 shows a cannula with a distal portion 121a and a proximal portion 122a comprising a collar. The cannula is inserted through an incision in the skin 120 of a patient. The distal portion 121a of the cannula comprises a time-of-flight emitter 124a of a time-of-flight distance sensor set. A distal portion 127b of a laparoscopic instrument is inserted through the access port of the cannula such that a proximal portion127a of the laparoscopic instrument is outside the cavity. At the distal portion 127a of the laparoscopic instrument, it comprises a time-of-flight receiver of the time-of-flight distance sensor set. The time-of-flight emitter 124a emits a spatially coherent light beam towards the surface area 125. At least a part of the light is reflected by the surface area 125 and received by the time-of-flight receiver at the distal portion 127a of the laparoscopic instrument and thus the distance between the laparoscopic instrument and the surface area 125 may be determined.

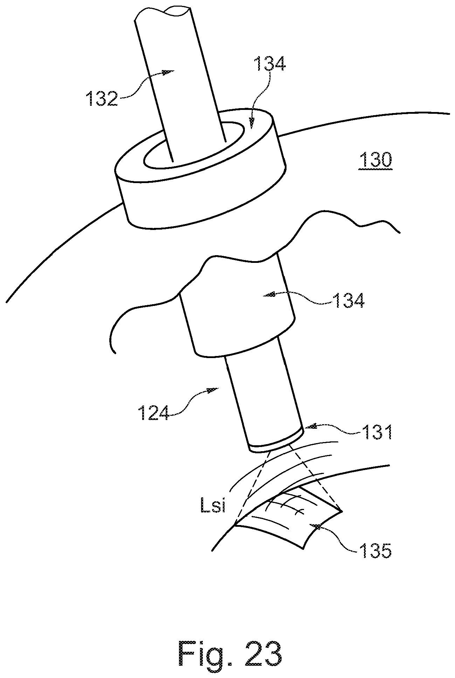

Fig. 23 shows a cannula 134 inserted through an incision in the skin 130 of a patient. A distal portion 131 of a laparoscopic instrument is inserted through the access port of the cannula such that a proximal part 132 of the laparoscopic instrument is outside the cavity. The distal portion 131 of the laparoscopic instrument comprises a not shown time-of-flight camera set 124 with an integrated spatial position tracking means. The time-of-flight camera set emits spatially incoherent light Lₛᵢ towards a surface area 135 and light is reflected back to the time-of-flight camera set.

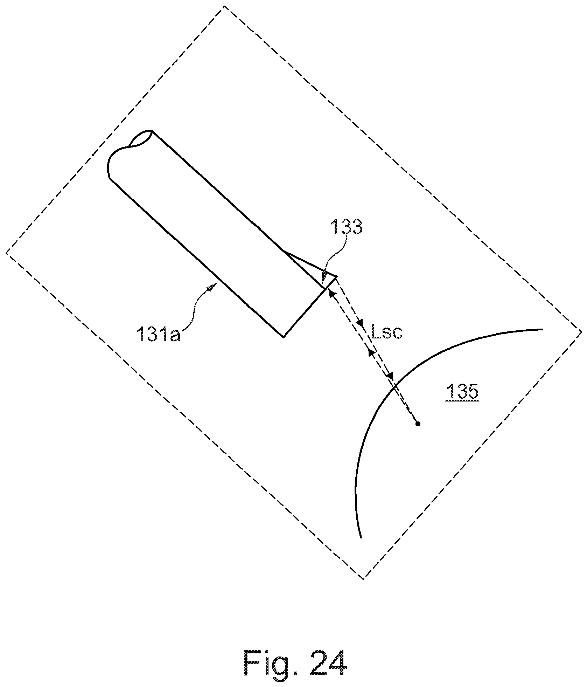

Fig. 24 shows a laparoscopic instrument with a distal portion 131a comprising a time-of-flight distance sensor set 133 emitting spatially coherent light L_{sc} towards a surface area 135 during a MIS procedure. At least a part of the light is reflected back to the time-of-flight distance sensor set 133. Thereby the distance between the laparoscopic instrument and the surface area 135 may be determined in real life fashion.

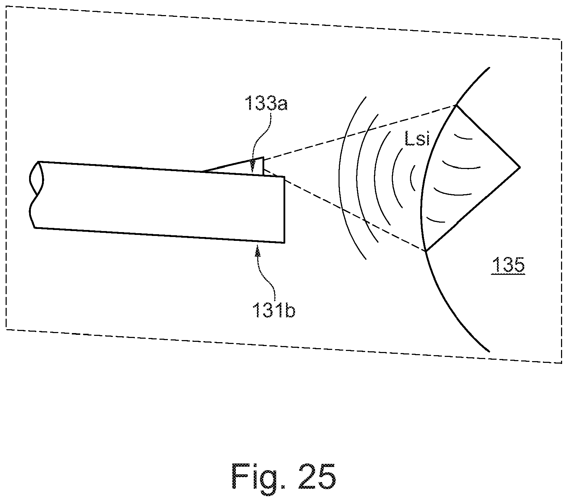

Fig. 25 shows a laparoscopic instrument with a distal portion 131b comprising a time-of-flight camera set 133a emitting spatially incoherent light Lₛᵢ towards a surface area 135 during a MIS procedure. At least a part of the light is reflected back to the time-of-flight camera set 133a.

The camera set 133a also comprises a spatial position tracking means e.g. in the form of the time-of-flight distance sensor set 133 of Fig. 24.

Thereby an associated computer system may calculate real time 3D images of the surface area 135.

## Claims

1. A laparoscopic tool system for minimally invasive surgery, said laparoscopic tool system comprises
• a laparoscopic tool (50, 70) comprising a proximal portion and a distal portion,
• a spatial position tracking means (56),
• a time-of-flight camera set, comprising a time-of-flight light emitter (53b, 53C, 107a) and a time-of-flight image sensor (53a, 53d) , and
• a computer system (54), said time-of-flight image sensor is fixed to said distal portion (21) of said laparoscopic tool (50), said spatial position tracking means (56) being adapted for tracking the spatial position of the time-of-flight image sensor (53a) and generating real time spatial position data of said time-of-flight image sensor (53a), said time-of-flight light emitter (53b) being configured for emitting light and said time-of-flight image sensor (53a) is configured for sensing light from said time-of-flight emitter (53b) and generating sensed light data when said emitted light is reflected from a surface area (55) within a preselected distance from said time-of-flight image sensor (53a), said computer system (54) being in data communication with said spatial position tracking means (56) and being configured for receiving real time spatial position data of said time-of-flight image sensor (53a), said computer system (54) being in data communication with said time-of-flight camera set and being configured for receiving said sensed light data timely corresponding to said real time spatial position data of said time-of-flight image sensor (53a), and said computer system (54) being configured for calculating a 3D image based on said sensed light data and real time spatial position data of said time-of-flight image sensor (53a), **characterized in that** wherein the spatial position tracking means (56) is adapted to tracking the spatial position of the time-of-flight image sensor (53a) relative to the time-of-flight light emitter (53b).

2. The laparoscopic tool system of claim 1, wherein the spatial position tracking means is configured for tracking the spatial position of the time-of-flight image sensor (71) relative to a fixed point and/or relative to an X,Y,Z coordinate matrix.

3. The laparoscopic tool system of any one of the preceding claims 1-2, wherein the spatial position tracking means comprises a motion sensor configured for determining motions of the laparoscopic tool, said spatial position tracking means is preferably configured for detecting acceleration.

4. The laparoscopic tool system of claim 3, wherein the laparoscopic tool forms part of or is mounted onto a robot for motorized maneuvering of said laparoscopic tool, said motion sensor being adapted for determining motions of the laparoscopic tool at least partly based on the motorized maneuvering of the laparoscopic tool.

5. The laparoscopic tool system of any one of the preceding claims 1-4, wherein the spatial position tracking means comprises at least one global position sensor or a local position sensor mounted to the distal portion of the laparoscopic tool.

6. The laparoscopic tool system of any one of the preceding claims 1-5, wherein the spatial position tracking means (81) is configured for detecting changes in rotational attributes, preferably comprising pitch, roll and yaw, preferably the spatial position tracking means comprises an inertial measurement system, such as an inertial measurement unit (IMU).

7. The laparoscopic tool system of any one of the preceding claims 1-6, wherein the spatial position tracking means comprises a structured light projecting unit mounted to the distal portion of the laparoscopic tool and adapted for projecting a structured light pattern (104), wherein the structured light pattern differs in wavelength(s) from one or more wavelengths emitted by the time-of-flight light emitter (107a).

8. The laparoscopic tool system of any one of the preceding claims 1-7, wherein the spatial position tracking means comprises a time-of-flight distance sensor set (112, 113) configured for determining a distance between the distal portion of the laparoscopic tool and a surface area, said time-of-flight distance sensor set, preferably comprises an further time-of-flight light emitter (112) and a further time-of-flight receiver (113t).

9. The laparoscopic tool system of any one of the preceding claims 1-8, wherein the time-of-flight light emitter (107a) is adapted to be located at a further laparoscopic tool (107) and the time-of-flight light emitter (107a) is adapted to be positioned closer to the surface to be analyzed than the time-of-flight image sensor, preferably the time-of-flight light emitter (107a) is positioned on a distal portion of the further laparoscopic tool.

10. The laparoscopic tool system of any one of the preceding claims 1-9, wherein the time-of-flight light emitter is adapted for emitting a spatially coherent light beam (Lsc), said emitter is optionally adapted
- for emitting a scanning beam or
- for emitting two or more stationary (non- scanning) beams for triangular determinations.

11. The laparoscopic tool system of any one of the preceding claims 1-10, wherein the time-of-flight image sensor is a camera comprising an array of pixel sensors each comprising a photodetector (such as an avalanche photodiode (APD), a photomultiplier or a metal-semiconductor-metal photodetector (MSM photodetector), preferably the time-of-flight image sensor comprises active pixel sensors (APS), preferably each pixel comprises an amplifier, more preferably the time-of-flight image sensor comprises at least about 1 kilo pixels, such as at least about 1 Mega pixels.

12. The laparoscopic tool system of any one of the preceding claims 1-11, wherein the time-of-flight image sensor is selected from a charge-coupled device (CDD) image sensor, or a complementary metal-oxide-semiconductor (CMOS) image sensor.

13. The laparoscopic tool system of any one of the preceding claims 1-12, wherein the time-of-flight image sensor is operatively connected to the time-of-flight light emitter and is configured for timely adjusting the emitter and the image sensor.

## Patentansprüche

1. Laparoskopisches Instrumentensystem für minimal-invasive Chirurgie, wobei das laparoskopische Instrumentensystem umfasst
• ein laparoskopisches Instrument (50, 70), das einen proximalen Abschnitt und einen distalen Abschnitt umfasst,
• ein Raumpositionsverfolgungsmittel (56),
• ein Laufzeitkameraset, das einen Laufzeitlichtsender (53b, 53c, 107a) und einen Laufzeitbildsensor (53a, 53d) umfasst, und
• ein Computersystem (54),
wobei der Laufzeitbildsensor an dem distalen Abschnitt (21) des laparoskopischen Instruments (50) befestigt ist, wobei das Raumpositionsverfolgungsmittel (56) zum Verfolgen der Raumposition des Laufzeitbildsensors (53a) und zum Erzeugen von Echtzeitraumpositionsdaten des Laufzeitbildsensors (53a) angepasst ist,
wobei der Laufzeitlichtsender (53b) zum Senden von Licht konfiguriert ist und der Laufzeitbildsensor (53a) zum Erfassen von Licht von dem Laufzeitsender (53b) und zum Erzeugen von erfassten Lichtdaten konfiguriert ist, wenn das gesendete Licht von einem Oberflächenbereich (55) innerhalb einer vorgewählten Entfernung von dem Laufzeitbildsensor (53a) reflektiert wird,
wobei das Computersystem (54) in Datenkommunikation mit dem Raumpositionsverfolgungsmittel (56) steht und zum Empfangen von Echtzeitraumpositionsdaten des Laufzeitbildsensors (53a) konfiguriert ist, wobei das Computersystem (54) in Datenkommunikation mit dem Laufzeitkameraset steht und zum Empfangen der erfassten Lichtdaten konfiguriert ist, die den Echtzeitraumpositionsdaten des Laufzeitbildsensors (53a) zeitlich entsprechen, und das Computersystem (54) zum Berechnen eines 3D-Bildes auf der Grundlage der erfassten Lichtdaten und der Echtzeitraumpositionsdaten des Laufzeitbildsensors (53a) konfiguriert ist, **dadurch gekennzeichnet, dass** wobei das Raumpositionsverfolgungsmittel (56) zum Verfolgen der Raumposition des Laufzeitbildsensors (53a) relativ zu dem Laufzeitlichtsender (53b) angepasst ist.

2. Laparoskopisches Instrumentensystem nach Anspruch 1, wobei das Raumpositionsverfolgungsmittel zum Verfolgen der Raumposition des Laufzeitbildsensors (71) relativ zu einem festen Punkt und/oder relativ zu einer X,Y,Z-Koordinatenmatrix konfiguriert ist.

3. Laparoskopisches Instrumentensystem nach einem der vorstehenden Ansprüche 1-2, wobei das Raumpositionsverfolgungsmittel einen Bewegungssensor umfasst, der zum Bestimmen von Bewegungen des laparoskopischen Instruments konfiguriert ist, wobei das Raumpositionsverfolgungsmittel vorzugsweise zum Erfassen von Beschleunigung konfiguriert ist.

4. Laparoskopisches Instrumentensystem nach Anspruch 3, wobei das laparoskopische Instrument einen Teil eines Roboters zum motorisierten Manövrieren des laparoskopischen Instruments bildet oder an einem solchen montiert ist, wobei der Bewegungssensor zum Bestimmen von Bewegungen des laparoskopischen Instruments mindestens teilweise auf der Grundlage des motorisierten Manövrierens des laparoskopischen Instruments angepasst ist.

5. Laparoskopisches Instrumentensystem nach einem der vorstehenden Ansprüche 1-4, wobei das Raumpositionsverfolgungsmittel mindestens einen globalen Positionssensor oder einen lokalen Positionssensor umfasst, der an dem distalen Abschnitt des laparoskopischen Instruments montiert ist.

6. Laparoskopisches Instrumentensystem nach einem der vorstehenden Ansprüche 1-5, wobei das Raumpositionsverfolgungsmittel (81) zum Erfassen von Änderungen von Rotationsattributen, die vorzugsweise Nicken, Rollen und Gieren umfassen, konfiguriert ist, wobei das Raumpositionsverfolgungsmittel vorzugsweise ein Inertialmesssystem, wie beispielsweise eine Inertialmesseinheit (IMU), umfasst.

7. Laparoskopisches Instrumentensystem nach einem der vorstehenden Ansprüche 1-6, wobei das Raumpositionsverfolgungsmittel eine Projektionseinheit für strukturiertes Licht umfasst, die an dem distalen Abschnitt des laparoskopischen Instruments montiert und zur Projektion eines strukturierten Lichtmusters (104) angepasst ist, wobei sich das strukturierte Lichtmuster in der/den Wellenlänge(n) von einer oder mehreren Wellenlängen unterscheidet, die von dem Laufzeitlichtsender (107a) gesendet werden.

8. Laparoskopisches Instrumentensystem nach einem der vorstehenden Ansprüche 1-7, wobei das Raumpositionsverfolgungsmittel einen Laufzeitentfernungssensorsatz (112, 113) umfasst, der zum Bestimmen einer Entfernung zwischen dem distalen Abschnitt des laparoskopischen Instruments und einem Oberflächenbereich konfiguriert ist, wobei der Laufzeitentfernungssensorsatz vorzugsweise einen weiteren Laufzeitlichtsender (112) und einen weiteren Laufzeitempfänger (113t) umfasst.

9. Laparoskopisches Instrumentensystem nach einem der vorstehenden Ansprüche 1-8, wobei der Laufzeitlichtsender (107a) angepasst ist, um an einem weiteren laparoskopischen Instrument (107) angeordnet zu werden, und der Laufzeitlichtsender (107a) angepasst ist, um näher an der zu analysierenden Oberfläche positioniert zu werden als der Laufzeitbildsensor, wobei der Laufzeitlichtsender (107a) an einem distalen Abschnitt des weiteren laparoskopischen Instruments positioniert ist.

10. Laparoskopisches Instrumentensystem nach einem der vorstehenden Ansprüche 1-9, wobei der Laufzeitlichtsender zum Senden eines räumlich kohärenten Lichtstrahls (L_{SC}) angepasst ist, wobei der Sender optional angepasst ist
- zum Senden eines scannenden Strahls oder
- zum Senden von zwei oder mehr stationären (nicht scannenden) Strahlen für Dreiecksbestimmungen.

11. Laparoskopisches Instrumentensystem nach einem der vorstehenden Ansprüche 1-10, wobei der Laufzeitbildsensor eine Kamera ist, die eine Anordnung von Pixelsensoren umfasst, die jeweils einen Photodetektor (wie beispielsweise eine Avalanche-Photodiode (APD), einen Photomultiplikator oder einen Metall-Halbleiter-Metall-Photodetektor (MSM-Photodetektor) umfassen, wobei der Laufzeitbildsensor vorzugsweise aktive Pixelsensoren (APS) umfasst, wobei vorzugsweise jedes Pixel einen Verstärker umfasst, wobei bevorzugter der Laufzeitbildsensor mindestens etwa 1 Kilopixel, wie beispielsweise mindestens etwa 1 Megapixel, umfasst.

12. Laparoskopisches Instrumentensystem nach einem der vorstehenden Ansprüche 1-11, wobei der Laufzeitbildsensor aus einem ladungsgekoppelte Vorrichtung- (CDD) Bildsensor oder einem komplementärer Metall-Oxid-Halbleiter- (CMOS) Bildsensor ausgewählt ist.

13. Laparoskopisches Instrumentensystem nach einem der vorstehenden Ansprüche 1-12, wobei der Laufzeitbildsensor betriebsmäßig mit dem Laufzeitlichtsender verbunden ist und zum zeitlichen Einstellen des Senders und des Bildsensors konfiguriert ist.

## Revendications

1. Système d'outil laparoscopique pour chirurgie mini-invasive, ledit système d'outil laparoscopique comprend
• un outil laparoscopique (50, 70), comprenant une partie proximale et une partie distale,
• un moyen de suivi (56) de position spatiale,
• un ensemble de caméra temps de vol, comprenant un émetteur de lumière temps de vol (53b, 53 C, 107a) et un capteur d'images temps de vol (53a, 53d), et
• un système informatique (54),
ledit capteur d'images temps de vol est fixé à ladite partie distale (21) dudit outil laparoscopique (50), ledit moyen de suivi (56) de position spatiale étant adapté pour suivre la position spatiale du capteur d'images temps de vol (53a) et engendrer des données de position spatiale en temps réel dudit capteur d'images temps de vol (53a),
ledit émetteur de lumière temps de vol (53b) étant configuré pour émettre de la lumière et ledit capteur d'images temps de vol (53a) configuré pour capter de la lumière provenant dudit émetteur temps de vol (53b) et engendrer des données de lumière captée lorsque ladite lumière émise est réfléchie par une zone superficielle (55) dans les limites d'une distance présélectionnée dudit capteur d'images temps de vol (53a),
ledit système informatique (54) étant en communication de données avec ledit moyen de suivi (56) de position spatiale et étant configuré pour recevoir des données de position spatiale en temps réel dudit capteur d'images temps de vol (53a), ledit système informatique (54) étant en communication de données avec ledit ensemble de caméra temps de vol et étant configuré pour recevoir lesdites données de lumière captée correspondant temporellement auxdites données de position spatiale en temps réel dudit capteur d'images temps de vol (53a), et ledit système informatique (54) étant configuré pour calculer une image 3D sur la base desdites données de lumière captée et données de position spatiale en temps réel dudit capteur d'images temps de vol (53a), **caractérisé en ce que** dans lequel le moyen de suivi (56) de position spatiale est adapté pour suivre la position spatiale du capteur d'images temps de vol (53a) par rapport à l'émetteur de lumière temps de vol (53b).

2. Système d'outil laparoscopique selon la revendication 1, dans lequel le moyen de suivi de position spatiale est configuré pour suivre la position spatiale du capteur d'images temps de vol (71) par rapport à un point déterminé et/ou par rapport à une matrice de coordonnées X, Y, Z.

3. Système d'outil laparoscopique selon l'une quelconque des revendications 1-2 précédentes, dans lequel le moyen de suivi de position spatiale comprend un capteur de mouvement configuré pour déterminer des mouvements de l'outil laparoscopique, ledit moyen de suivi de position spatiale est de préférence configuré pour détecter une accélération.

4. Système d'outil laparoscopique selon la revendication 3, dans lequel l'outil laparoscopique fait partie d'un ou est monté sur un robot destiné à la manœuvre motorisée dudit outil laparoscopique, ledit capteur de mouvement étant adapté pour déterminer des mouvements de l'outil laparoscopique au moins en partie sur la base de la manœuvre motorisée de l'outil laparoscopique.

5. Système d'outil laparoscopique selon l'une quelconque des revendications 1-4 précédentes, dans lequel le moyen de suivi de position spatiale comprend au moins un capteur de position globale ou un capteur de position locale monté sur la partie distale de l'outil laparoscopique.

6. Système d'outil laparoscopique selon l'une quelconque des revendications 1-5 précédentes, dans lequel le moyen de suivi (81) de position spatiale est configuré pour détecter des changements dans des attributs de rotation, comprenant de préférence tangage, roulis et lacet, de préférence le moyen de suivi de position spatiale comprend un système de mesure inertiel, tel qu'une unité de mesure inertielle (IMU).

7. Système d'outil laparoscopique selon l'une quelconque des revendications 1-6 précédentes, dans lequel le moyen de suivi de position spatiale comprend une unité de projection de lumière structurée montée sur la partie distale de l'outil laparoscopique et adaptée pour projeter un motif (104) de lumière structurée, le motif de lumière structurée différant en longueur(s) d'onde d'une ou de plusieurs longueurs d'onde émises par l'émetteur de lumière temps de vol (107a).

8. Système d'outil laparoscopique selon l'une quelconque des revendications 1-7 précédentes, dans lequel le moyen de suivi de position spatiale comprend un ensemble de capteur de distance temps de vol (112,113) configuré pour déterminer une distance entre la partie distale de l'outil laparoscopique et une zone superficielle, ledit ensemble de capteur de distance temps de vol comprend de préférence un autre émetteur de lumière temps de vol (112) et un autre récepteur temps de vol (113t).

9. Système d'outil laparoscopique selon l'une quelconque des revendications 1-8 précédentes, dans lequel l'émetteur de lumière temps de vol (107a) est adapté pour être situé sur un autre outil laparoscopique (107) et l'émetteur de lumière temps de vol (107a) est adapté pour être positionné plus près de la surface à analyser que le capteur d'images temps de vol, de préférence l'émetteur de lumière temps de vol (107a) est positionné sur une partie distale de l'autre outil laparoscopique.

10. Système d'outil laparoscopique selon l'une quelconque des revendications 1-9 précédentes, dans lequel l'émetteur de lumière temps de vol est adapté pour émettre un faisceau de lumière spatialement cohérente (Lsc), ledit émetteur est en option adapté
- pour émettre un faisceau de balayage ou
- pour émettre deux ou plus de deux faisceaux stationnaires (non de balayage) pour des déterminations triangulaires.

11. Système d'outil laparoscopique selon l'une quelconque des revendications 1-10 précédentes, dans lequel le capteur d'images temps de vol est une caméra comprenant un groupement de capteurs de pixels comprenant chacun un photodétecteur (tel qu'une photodiode à avalanche (APD), un photomultiplicateur ou un photodétecteur métal-semiconducteur-métal (photodétecteur MSM), de préférence le capteur d'images temps de vol comprend des capteurs à pixels actifs (APS), de préférence chaque pixel comprend un amplificateur, de façon plus particulièrement préférée le capteur d'images temps de vol comprend au moins environ 1 kilopixel, comme au moins environ 1 mégapixel.

12. Système d'outil laparoscopique selon l'une quelconque des revendications 1-11 précédentes, dans lequel le capteur d'images temps de vol est choisi parmi un capteur d'images à dispositif à couplage de charges (CDD), ou un capteur d'images à semiconducteur à oxyde métallique complémentaire (CMOS).

13. Système d'outil laparoscopique selon l'une quelconque des revendications 1-12 précédentes, dans lequel le capteur d'images temps de vol est connecté de façon opérationnelle à l'émetteur de lumière temps de vol et est configuré pour ajuster temporellement l'émetteur et le capteur d'images.
